# EUROPEAN PATENT APPLICATION

(11) **EP 0 934 919 A1**
(43) Date of publication of application: **11.08.1999**
(21) Application number: 97941230.1
(22) Date of filing: 25.09.1997
(51) Int. Cl.: C07C 25/18, C07C 43/225, C09K 19/10, C09K 19/42, G02F 1/13

(54) **SUBSTITUTED BENZENE DERIVATIVE, LIQUID-CRYSTAL COMPOSITION, AND LIQUID-CRYSTAL DISPLAY ELEMENT**

(30) Priority: 25.09.1996 JP 27285796
(71) Applicant: CHISSO CORPORATION, Osaka-shi Osaka 530-6591 (JP)
(72) Inventor: KONDOU, Tomoyuki, Ichihara-shi, Chiba-ken 290 (JP); MATSUI, Shuichi, Ichihara-shi, Chiba-ken 290 (JP); MIYAZAWA, Kazutoshi, Yachiyo-shi, Chiba (JP); TAKEUCHI, Hiroyuki, Ichihara-shi, Chiba (JP); KUBO, Yasuhiro, Ichihara-shi, Chiba-ken 290 (JP); TAKESHITA, Fusayuki, Sodegaura-shi, Chiba (JP); NAKAGAWA, Etsuo, Ichihara-shi, Chiba (JP)
(74) Representative: HOFFMANN - EITLE
(86) International application number: JP9703403
(87) International publication number: WO9813323

(57) **Abstract**

There is provided a liquid crystalline compound having a high voltage holding ratio which has a very small degree of temperature dependence, a low threshold voltage which has a very small degree of temperature dependence, a high Δn, and excellent miscibility with other liquid crystal materials at low temperature; a liquid crystal composition; and a liquid crystal display element, and the liquid crystalline compound is a substituted benzene derivative represented by general formula (1), 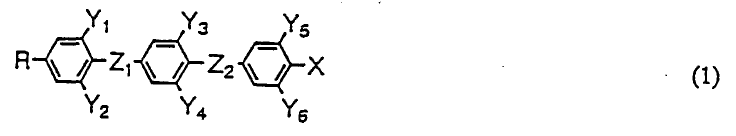 where, R represents a straight chain or branched alkyl group having 1 to 20 carbon atoms in which each of optional and nonadjacent methylene groups (-CH₂-) may be substituted by an oxygen atom; X represents a halogen atom, -CF₃, -CF₂H, -CFH₂, -OCF₃ or -OCF₂H; each of Z₁ and Z₂ independently represents -(CH₂)₂-, -(CH₂)₄-, -CH₂O-, -OCH₂- or a covalent bond, but Z₁ and Z₂ are not covalent bonds simultaneously; each of Y₁, Y₂, Y₃, Y₄, Y₅, and Y₆ independently represents H or F, but at least one of Y₁, Y₂, Y₃, and Y₄ represents F; and any atom constituting this compound may be substituted by an isomer thereof.

## Description

### Technical Field

The present invention relates to a novel liquid crystalline compound and a liquid crystal composition. In particular, the present invention relates to a compound having fluorine-substituted 1,4-phenylene groups, a liquid crystal composition containing such a compound, and a liquid crystal display element constituted from such a liquid crystal composition.

### Background Art

Display elements produced from liquid crystalline compounds have been used widely in displays for watches and clocks, electronic calculators, word processors, and the like. (The term "liquid crystalline compound" used herein is a generic designation for compounds that exhibit a liquid crystal phase, as well as compounds that do not exhibit a liquid crystal phase but are useful as constituting components for liquid crystal compositions.) In recent years, TFT displays having characteristics such as high contrast and wide viewing angle have been actively studied.

For liquid crystal compositions for TFT displays, there are demanded such properties as a high voltage holding ratio which has a very small degree of temperature dependence, a low threshold voltage (Vth) which has a very small degree of temperature dependence, wide range of mesophase, excellent miscibility with other liquid crystal materials, and low viscosity. Also, compositions having a high Δn are useful for improving response speed.

Fluorine-substituted liquid crystalline compounds are suitable as components constituting liquid crystal compositions having such properties, and a large number of such compounds have been examined as described in (1) Japanese Patent Publication No. 63-13411, (2) Japanese Patent Publication No. 63-44132, (3) Japanese Patent Application Laid open No. 2-233626, (4) Japanese-translated PCT Patent Application Laid-open No. 2-501311, (5) Japanese-translated PCT Patent Application Laid-open No. 3-500413, (6) Japanese-translated PCT Patent Application Laid-open No. 3-503771, (7) Japanese-translated PCT Patent Application Laid-open No. 3-504018, (8) Japanese Patent Application Laid Open No. 4-217930, (9) Japanese-translated PCT Patent Application Laid-open No. 4-501575, (10) Japanese-translated PCT Patent Application Laid-open No. 5-502676, (11) Japanese-translated PCT Patent Application Laid-open No. 6-504032, and (12) EP 436089.

### Disclosure of Invention

An object of the present invention is to provide a liquid crystalline compound having a high voltage holding ratio which has a very small degree of temperature dependence, a low threshold voltage which has a very small degree of temperature dependence, a high Δn, and excellent miscibility with other liquid crystal materials at low temperature; liquid crystal compositions produced from these compounds; and liquid crystal display elements constituted from such liquid crystal compositions. The present inventors found that the above object is achieved by substituted benzene derivatives represented by general formula (1), thus completing the present invention. 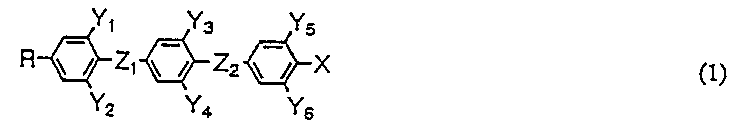 where, R represents a straight chain or branched alkyl group having 1 to 20 carbon atoms, in which each of optional and nonadjacent methylene groups (-CH₂-) may be substituted by an oxygen atom; X represents a halogen atom, -CF₃, -CF₂H, -CFH₂, -OCF₃ or -OCF₂H; each of Z₁ and Z₂ independently represents -(CH₂)₂-, -(CH₂)₄-, -CH₂O-, - OCH₂- or a covalent bond, but Z₁ and Z₂ are not covalent bonds simultaneously; each of Y₁, Y₂, Y₃, Y₄, Y₅ and Y₆ independently represents H or F, but at least one of Y₁, Y₂, Y₃ and Y₄ represents F; and,
1) when Z₁ = -(CH₂)₂-, Z₂ = a covalent bond, and
   a) when Y₁ = F and Y₂ = Y₃ = Y₄ = H,
      Y₅ = F,
   b) when Y₃ = F and Y₁ = Y₂ = Y₄ = H,
      Y₅ = Y₆ = F,
   c) when Y₁ = Y₂ = F, Y₃ = Y₄ = H and X = F or -CF₃,
      Y₅ = F,
   d) when Y₁ = Y₂ = F, Y₃ = Y₄ = H and X = -CF₂H,
      Y₆ = H,
   e) when Y₁ = Y₃ = F and Y₂ = Y₄ = H,
      Y₅ = Y₆ = F or Y₅ = Y₆ = H,
   f) when Y₃ = Y₄ = F, Y₁ = Y₂ = H and X = -OCF₃ or -OCF₂H,
      Y₅ = F,
   g) when Y₃ = Y₄ = F, Y₁ = Y₂ = H and X = -CF₃ or -CF₂H,
      Y₅ = Y₆ = F,
   h) when Y₁ = Y₂ = Y₃ = F, Y₄ = H and X = F, -OCF₃, -OCF₂H, -CF₃ or -CF₂H,
      Y₆ = H,
   i) when Y₁ = Y₂ = Y₃ = F, Y₄ = H and X = Cl,
      Y₅ = F,
   j) when Y₁ = Y₃ = Y₄ = F, Y₂ = H and X = F,
      Y₆ = F,
   k) when Y₁ = Y₂ = Y₃ = Y₄ = F and X = F, -OCF₃, -OCF₂H, -CF₃ or -CF₂H,
      Y₆ = H, and
   l) when Y₁ = Y₂ = Y₃ = Y₄ = F and X = Cl,
      Y₅ = F,
2) when Z₁ = a covalent bond, Z₂ = -(CH₂)₂-, and
   m) when Y₁ = F and Y₂ = Y₃ = Y₄ = H,
      Y₅ = F and Y₆ = H,
   n) when Y₃ = F and Y₁ = Y₂ = Y₄ = H,
      Y₅ = Y₆ = F or Y₅ = Y₆ = H,
   o) when Y₁ = Y₃ = F and Y₂ = Y₄ = H,
      Y₅ = Y₆ = F or Y₅ = Y₆ = H,
   p) when Y₃ = Y₄ = F, Y₁ = Y₂ = H and X = F, Cl, -OCF₃, -OCF₂H or -CF₂H,
      Y₆ = H,
   q) when Y₁ = Y₃ = Y₄ = F and Y₂ = H,
      Y₆ = H,
   r) when Y₁ = Y₂ = Y₃ = Y₄ = F,
      Y₆ = H,
with the proviso that when Z₁ = -(CH₂)₂-, Z₂ = a covalent bond, Y₃ = Y₄ = F and Y₁ = Y₂ = H, X is neither F nor Cl; when Z₁ is a covalent bond, Z₂ is -(CH₂)₂-, Y₁ = F and Y₂ = Y₃ = Y₄ = H, X is neither F, Cl nor CF₃; and any atom constituting this compound may be substituted by an isomer thereof.

Although some of the compounds represented by general formula (1) disclosed herein are included only perfunctorily in the above references (6) through (12) and other references, these references provide neither data on the compounds of the present invention nor specific descriptions of properties thereof, nor do they suggest the present invention.

Compounds represented by general formula (1) are classified into the following (a-1) through (a-32), and (b-1) through (b-32):

R-B(F)-(CH₂)₂-B-Q (a-1) R-B-(CH₂)₂-B(F)-Q (a-2) R-B(F,F)-(CH₂)₂-B-Q (a-3) R-B(F)-(CH₂)₂-B(F)-Q (a-4) R-B-(CH₂)₂-B(F,F)-Q (a-5) R-B(F,F)-(CH₂)₂-B(F)-Q (a-6) R-B(F)-(CH₂)₂-B(F,F)-Q (a-7) R-B(F,F)-(CH₂)₂-B(F,F)-Q (a-8) R-B(F)-(CH₂)₄-B-Q (a-9) R-B-(CH₂)₄-B(F)-Q (a-10) R-B(F,F)-(CH₂)₄-B-Q (a-11) R-B(F)-(CH₂)₄-B(F)-Q (a-12) R-B-(CH₂)₄-B(F,F)-Q (a-13) R-B(F,F)-(CH₂)₄-B(F)-Q (a-14) R-B(F)-(CH₂)₄-B(F,F)-Q (a-15) R-B(F,F)-(CH₂)₄-B(F,F)-Q (a-16) R-B(F)-CH₂O-B-Q (a-17) R-B-CH₂O-B(F)-Q (a-18) R-B(F,F)-CH₂O-B-Q (a-19) R-B(F)-CH₂O-B(F)-Q (a-20) R-B-CH₂O-B(F,F)-Q (a-21) R-B(F,F)-CH₂O-B(F)-Q (a-22)

R-B(F)-CH₂O-B(F,F)-Q (a-23) R-B(F,F)-CH₂O-B(F,F)-Q (a-24) R-B(F)-OCH₂-B-Q (a-25) R-B-OCH₂-B(F)-Q (a-26) R-B(F,F)-OCH₂-B-Q (a-27) R-B(F)-OCH₂-B(F)-Q (a-28) R-B-OCH₂-B(F,F)-Q (a-29) R-B(F,F)-OCH₂-B(F)-Q (a-30) R-B(F)-OCH₂-B(F,F)-Q (a-31) R-B(F,F)-OCH₂-B(F,F)-Q (a-32) R-B(F)-B-(CH₂)₂-Q (b-1) R-B-B(F)-(CH₂)₂-Q (b-2) R-B(F,F)-B-(CH₂)₂-Q (b-3) R-B(F)-B(F)-(CH₂)₂-Q (b-4) R-B-B(F,F)-(CH₂)₂-Q (b-5) R-B(F,F)-B(F)-(CH₂)₂-Q (b-6) R-B(F)-B(F,F)-(CH₂)₂-Q (b-7) R-B(F,F)-B(F,F)-(CH₂)₂-Q (b-8) R-B(F)-B-(CH₂)₄-Q (b-9) R-B-B(F)-(CH₂)₄-Q (b-10) R-B(F,F)-B-(CH₂)₄-Q (b-11) R-B(F)-B(F)-(CH₂)₄-Q (b-12) R-B-B(F,F)-(CH₂)₄-Q (b-13) R-B(F,F)-B(F)-(CH₂)₄-Q (b-14) R-B(F)-B(F,F)-(CH₂)₄-Q (b-15)

R-B(F,F)-B(F,F)-(CH₂)₄-Q (b-16) R-B(F)-B-CH₂O-Q (b-17) R-B-B(F)-CH₂O-Q (b-18) R-B(F,F)-B-CH₂O-Q (b-19) R-B(F)-B(F)-CH₂O-Q (b-20) R-B-B(F,F)-CH₂O-Q (b-21) R-B(F,F)-B(F)-CH₂O-Q (b-22) R-B(F)-B(F,F)-CH₂O-Q (b-23) R-B(F,F)-B(F,F)-CH₂O-Q (b-24) R-B(F)-B-OCH₂-Q (b-25) R-B-B(F)-OCH₂-Q (b-26) R-B(F,F)-B-OCH₂-Q (b-27) R-B(F)-B(F)-OCH₂-Q (b-28) R-B-B(F,F)-OCH₂-Q (b-29) R-B(F,F)-B(F)-OCH₂-Q (b-30) R-B(F)-B(F,F)-OCH₂-Q (b-31) R-B(F,F)-B(F,F)-OCH₂-Q (b-32)

In these formulas, R has the same meaning as described above; B represents a 1,4-phenylene group; B(F) represents a 3-fluoro-1, 4-phenylene group; B(F,F) represents a 3,5-difluoro-1,4-phenylene group; and Q represents the group shown below. 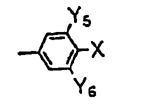 Where Y₅, Y₆, and X have the same meanings as described above.

Although compounds represented by the above formulas (a-1) through (a-32), and (b-1) through (b-32) are all preferred, compounds represented by formulas (a-1) through (a-8), (a-17) through (a-24), (b-1) through (b-8), and (b-17) through (b-24) are particularly preferred.

In these formulas, R represents a straight-chain or branched alkyl group having 1 to 20 carbon atoms specifically exemplified by methyl, ethyl, propyl, butyl, pentyl, hexyl, heptyl, decyl, pentadecyl, and icosyl groups as a straight-chain alkyl group; and isopropyl, sec-butyl, tert-butyl, 2-methylbutyl, isopentyl, isohexyl, 3-ethyloctyl, 3,8-dimethyltetradecyl, and 5-ethyl-5-methylnonadecyl groups as a branched alkyl group. The branched alkyl group may be the one having optical activity, and such a group is useful as a chiral doping agent.

Each of optional, nonadjacent methylene groups may be substituted by an oxygen atom, specifically exemplified by an alkoxyl group such as methoxy, ethoxy, propoxy, butoxy, pentyloxy, and nonyloxy groups; and an alkoxyalkyl group such as methoxymethyl, methoxyethyl, methoxypropyl, methoxybutyl, methoxypentyl, methoxyoctyl, ethoxymethyl, ethoxyethyl, ethoxypropyl, ethoxyhexyl, propoxymethyl, propoxyethyl, propoxypropyl, propoxypentyl, butoxymethyl, butoxyethyl, butoxybutyl, pentyloxymethyl, pentyloxybutyl, hexyloxymethyl, hexyloxyethyl, hexyloxypropyl, heptyloxymethyl, and octyloxymethyl groups.

In general formula (1), each of Z₁ and Z₂ independently represents -(CH₂)₂-, -(CH₂)₄-, -CH₂O-, -OCH₂-, or a covalent bond, and preferably, one of Z₁ and Z₂ is a covalent bond, and more preferably, one of Z₁ and Z₂ is a covalent bond and the other is -(CH₂)₂-, -CH₂O-, or -OCH₂-.

Any atom constituting the compound represented by formula (1) may be substituted by an isomer thereof.

By suitable selection of these substituents or bonding groups, a compound having desired properties can be obtained. A liquid crystalline compound according to the present invention represented by general formula (1) can be produced through commonly employed organic synthesis methods. For example, such a compound can be produced easily by the following method: 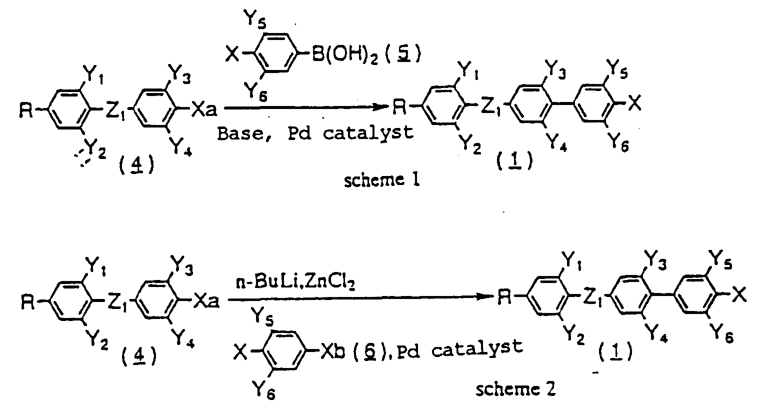 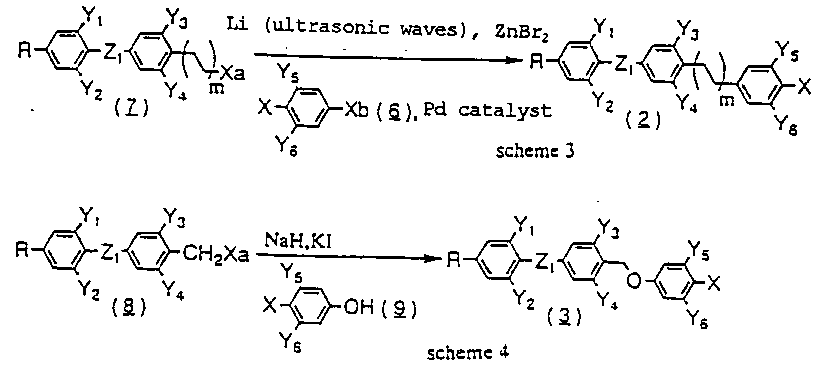 where R, X, Y₁ to Y₆, and Z₁ have the same meanings as described above; each of Xa and Xb represents a halogen atom; and m represents 1 or 2.

As is shown in Scheme 1, compound (1) of the present invention can be produced by allowing a halogen compound (4) to react with a dihydroxyborane derivative (5) in a mixed solvent containing toluene or xylene, an alcohol such as ethanol, and water, in the presence of a base such as K₂CO₃ or Na₂CO₃ and a catalyst such as palladium supported by graphite carbon (Pd-C), Pd(PPh₃)₄, or PdCl₂(PPh₃)₂. Alternatively, as Scheme 2 shows, compound (1) of the present invention can be produced by allowing a halogen compound (4) to react with a Lithium compound such as n-BuLi and a zinc compound such as ZnCl₂ or ZnBr₂, and then with a halogen compound (6).

As Scheme 3 shows, the compound (2) of the present invention can be produced by allowing a halogen compound (7) to react with lithium, then with a zinc compound and a halogen compound (6).

Also, as Scheme 4 shows, the compound (3) of the present invention can be produced by allowing a halogen compound (8) to react with a phenol derivative (9) in a solvent such as dimethyl sulphoxide, dimethyl formamide (DMF), 1,2-dimethoxyethane, tetrahydrofuran, hexamethylphosphorous triamide, or toluene, in the presence of a base such as sodium amide (J. B. Wright, et al., Journal of the American Chemical Society, 70, 3098(1948)), potassium carbonate (W. T. Olson, et al., Journal of the American Chemical Society, 69, 2451(1947)), triethyl amine (R. L. Merker, et al., the Journal of Organic Chemistry, 26, 5180(1961)), sodium hydroxide (C. Wilkins, Synthesis, 1973, 156), potassium hydroxide (J. Rebek, et al., the Journal of Organic Chemistry, 44, 1485(1979)), barium hydroxide (Kawabe, et al., the Journal of Organic Chemistry, 37, 4210(1972)), or sodium hydride (C. J. Stark, Tetrahedron Letters, 22, 2089, (1981), and K. Takai, et al., Tetrahedron Letters, 21, 1657, (1980)).

In general formula (1), a compound containing -O- in R can also be produced by similar methods.

The substituent X can be easily introduced by use of a previously introduced material, or through a well-known reaction at any process stage. Specific examples are shown below. In the formulas, Rx represents the following group: 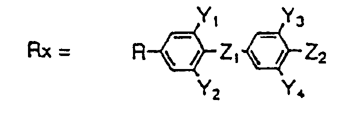 where R, Y₁ to Y₄, Z₁, and Z₂ have the same meanings as described above. 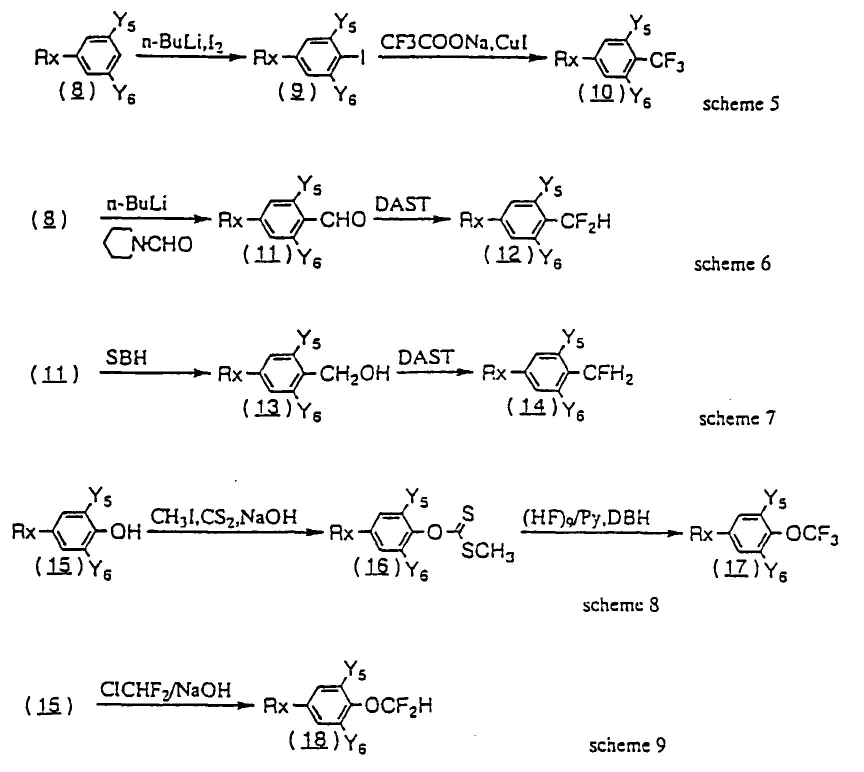 where Y₅ and Y₆ have the same meanings as described above.

As Scheme 5 shows, a trifluoromethyl compound (10) can be produced by allowing compound (8) to react with a lithium compound such as n-butyl lithium and iodine to form compound (9), and allowing compound (9) to react with sodium trifluoroacetate/copper(I) iodide (G. E. Carr, et al., Journal of the Chemical Society Parkin Transactions I, 921, (1988)), or methyl fluorosulfonyl difluoroacetate/copper(I) iodide (Q. Y. Chen, et al., Journal of the Chemical Society Chemical Communications, 705 (1989)).

As Scheme 6 shows, a difluoromethyl compound (12) can be produced by allowing compound (8) to react with a lithium compound such as n-butyl lithium and a formylating agent such as N-formyl piperidine (G. A. Olah, et al., Angewandte Chemie International Edition in English, 20, 878(1981)), N-formyl morpholine (G. A. Olah, et al., the Journal of Organic Chemistry, 49, 385(1984)), or DMF (G. Boss, et al., Chemische Berichte, 1199(1989)) to form compound (11), and allowing compound (11) to react with a fluorinating agent such as diethylaminosulfur trifluoride (DAST) (W. J. Middleton, et al., the Journal of Organic Chemistry, 40, 574(1975); S. Rozen, et al., Tetrahedron Letters, 41, 111(1985); M.Hudlicky, Organic Reactions, 35, 513(1988); and P. A. Messina, et al., the Journal of Fluorine Chemistry, 42, 137(1989)), or morpholinosulfur trifluoride (K. C. Mange, et al., the Journal of Fluorine Chemistry, 43, 405(1989)).

As Scheme 7 shows, a monofluoromethyl compound (14) can be produced by reducing compound (11) with a reductant such as sodium borohydride (SBH), lithium aluminum hydride (LAH), diisobutyl aluminum hydride (DIBAL), or sodium bis-(2-methoxyethoxy)-aluminum hydride (SBMEA) to form compound (13), and allowing compound (13) to react with a fluorinating agent such as DAST.

As Scheme 8 shows, a trifluoromethoxy compound (17) can be produced by converting compound (15) by use of the method of Albert, et al. (Synthetic Communications, 19, 547(1989)) to form compound (16), and fluorinating compound (16) through the method of Kuroboshi, et al. (Tetrahedron Letters, 33, 29, 4173(1992)).

As Scheme 9 shows, a difluoromethoxy compound (18) can be produced by fluorinating compound (15) in a chlorodifluoromethane/sodium hydroxide system (Japanese-translated PCT Patent Application Laid-open No. 3-500413). Alternatively, it can be produced by use of the method of Chen, et al. (the Journal of Fluorine Chemistry, 44, 433(1989).

Halogen compounds and dihydroxyborane derivatives used as the materials can be produced by general organic synthesis methods, such as the following schemes: 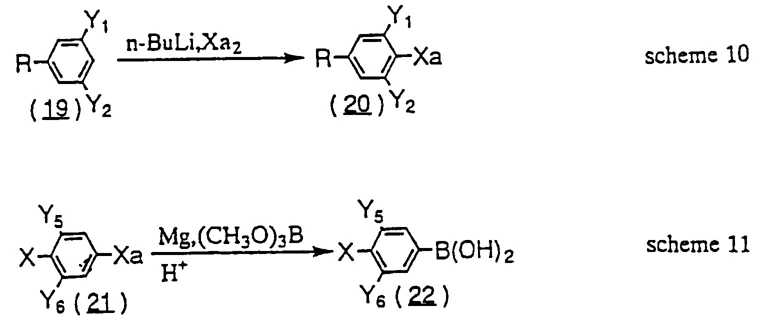 Where, R, X, Xa, Y₁, Y₂, Y₅, and Y₆ have the same meanings as described above.

As Scheme 10 shows, a halogen compound (20) can be produced by allowing compound (19) to react with a lithium compound such as n-BuLi, and iodine or bromine.

Also, as Scheme 11 shows, a dihydroxyborane derivative (22) can be produced by allowing compound (21) to react with a Grignard reagent prepared from and magnesium and a borane derivative such as trimethoxyborane or triisopropyloxyborane, and hydrolyzing the reaction product with hydrochloric acid or the like.

The reactions described above are well known to the art, and, needless to say, other known reactions may also be used.

Liquid crystalline compounds of the present invention thus obtained have a high voltage holding ratio which has a very small degree of temperature dependence, a low threshold voltage which has a very small degree of temperature dependence, and a high Δn. In addition, these compounds are easily mixed with various liquid crystal materials, and have high solubility even at low temperature.

Also, the liquid crystalline compounds of the present invention are physically and chemically stable under conditions where liquid crystal display elements are normally used, and are excellent materials for the components of nematic liquid crystal compositions.

The compounds of the present invention can also be suitably, used as the components of liquid compositions for TN, STN, and TFT displays.

The liquid crystal composition of the present invention will be described in detail below. Preferably, the liquid crystal composition according to the present invention contains at least one of the compounds represented by general formula (1) in a quantity of 0.1 to 99.9% by weight.

Specifically, liquid crystal compositions provided by the present invention are produced by mixing the first component containing at least one of the compounds represented by general formula (1), with a compound selected from a group of compounds represented by general formulas (2) to (9), depending on the application of the liquid crystal composition.

Preferred examples of compounds represented by general formulas (2) to (4) used in the liquid crystal composition of the present invention are as follows: 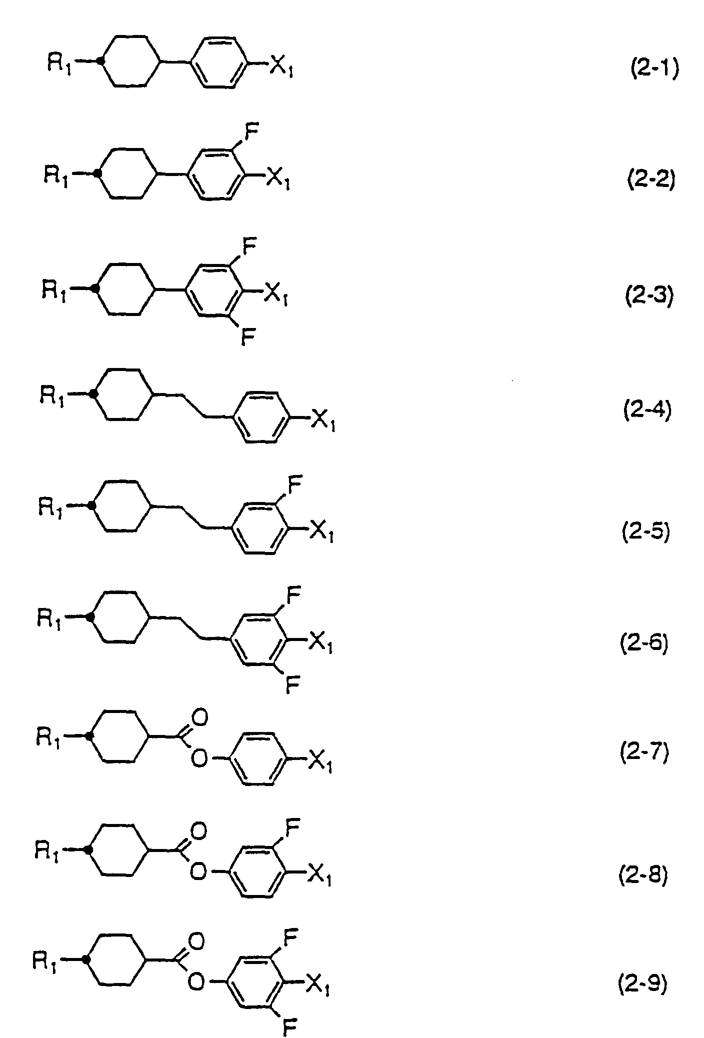 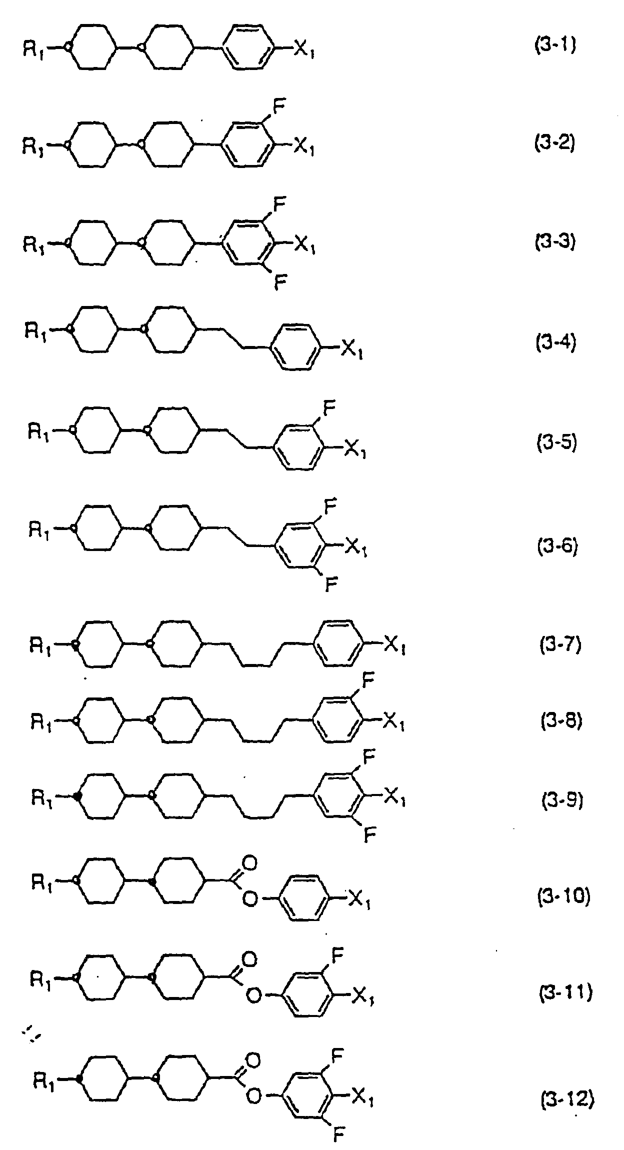 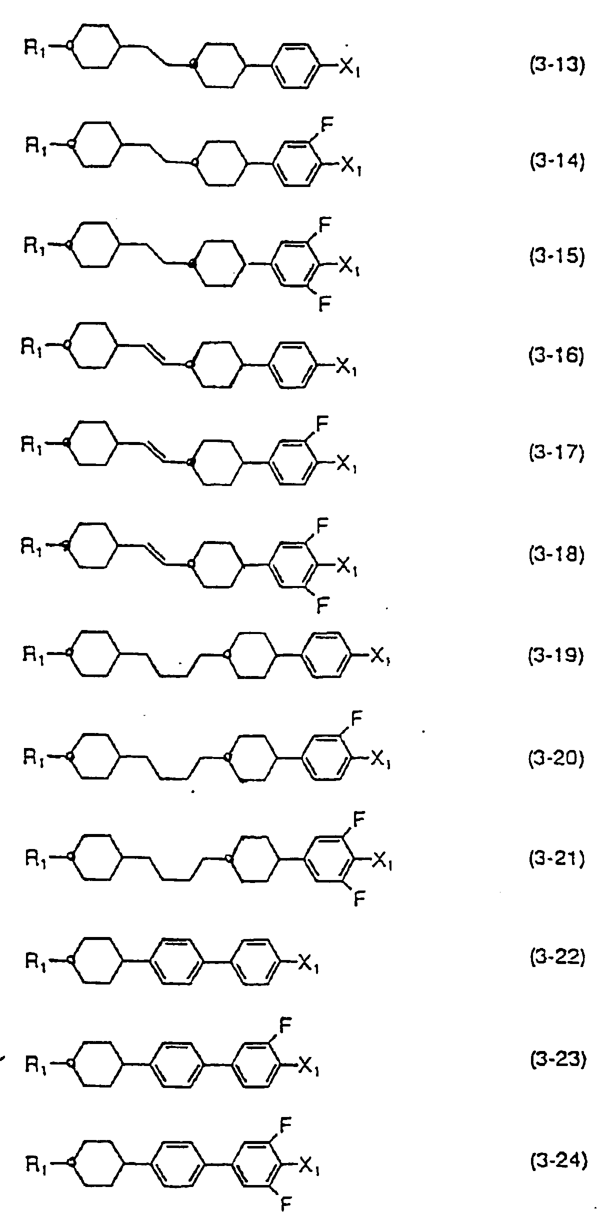 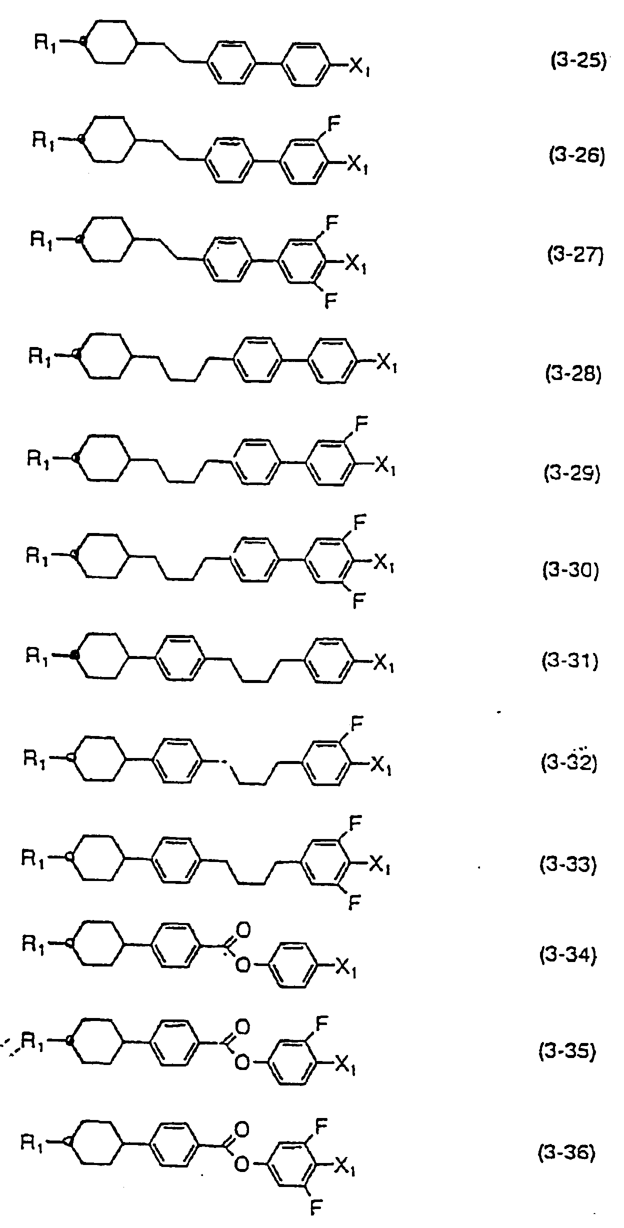 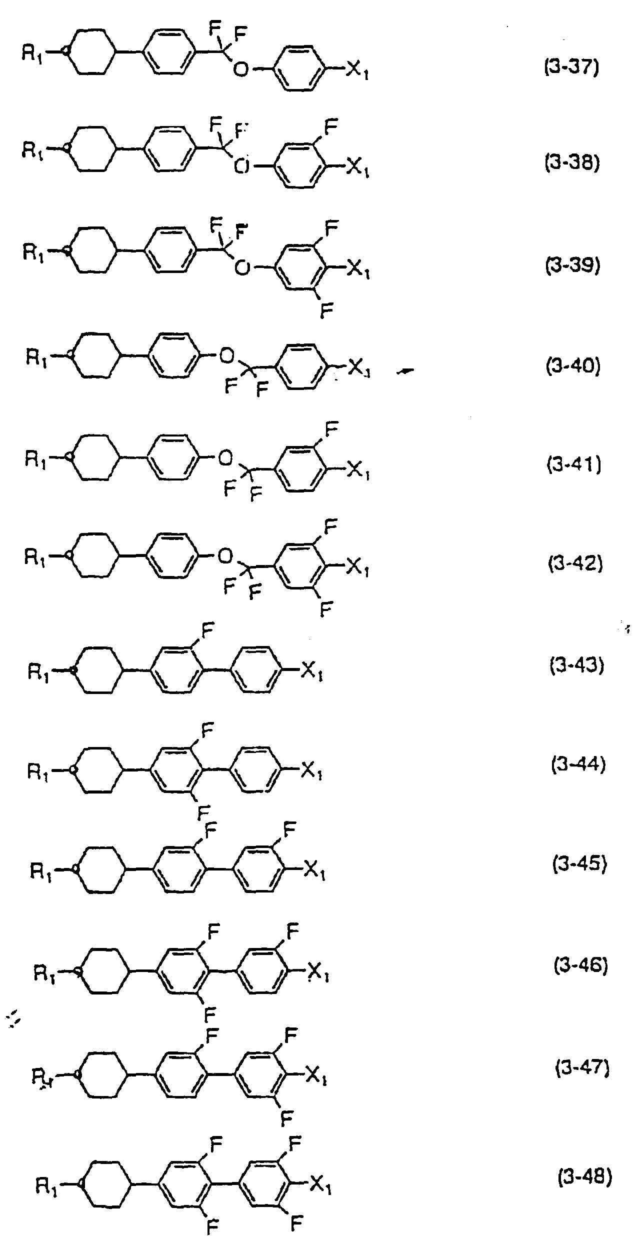 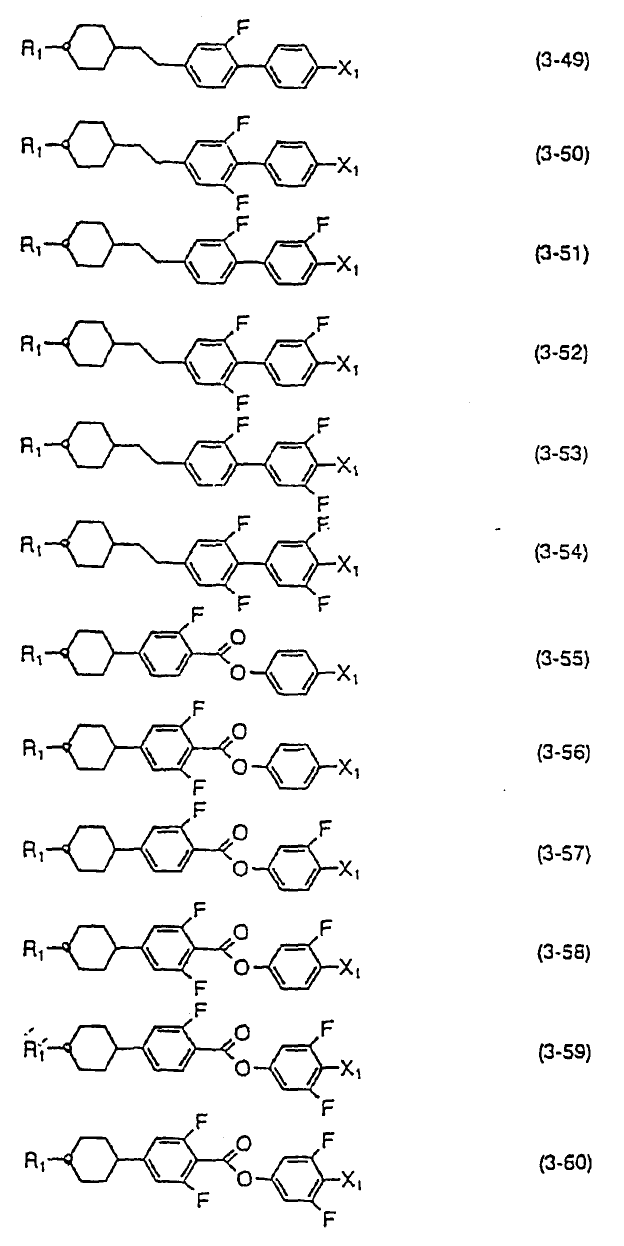 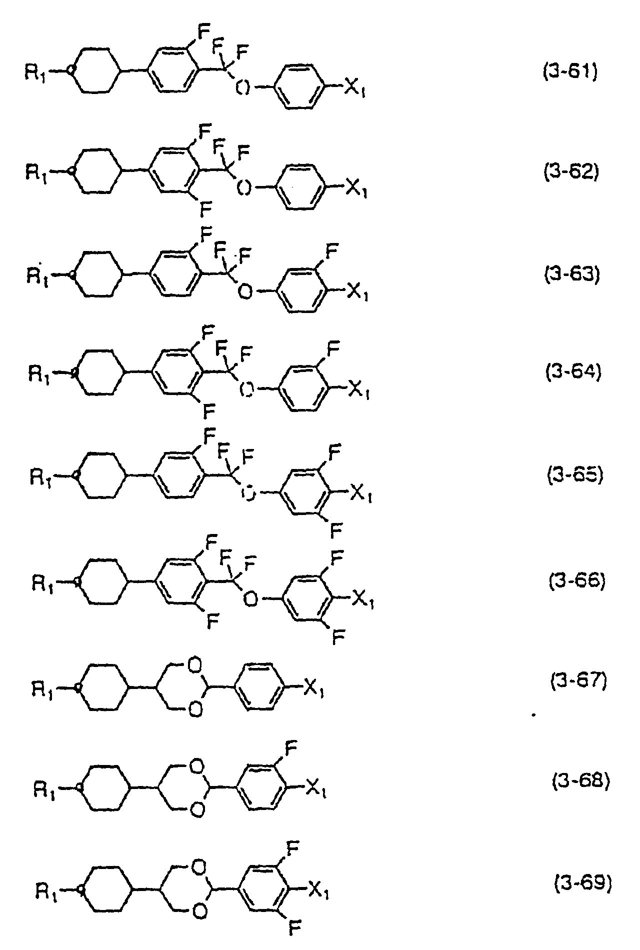 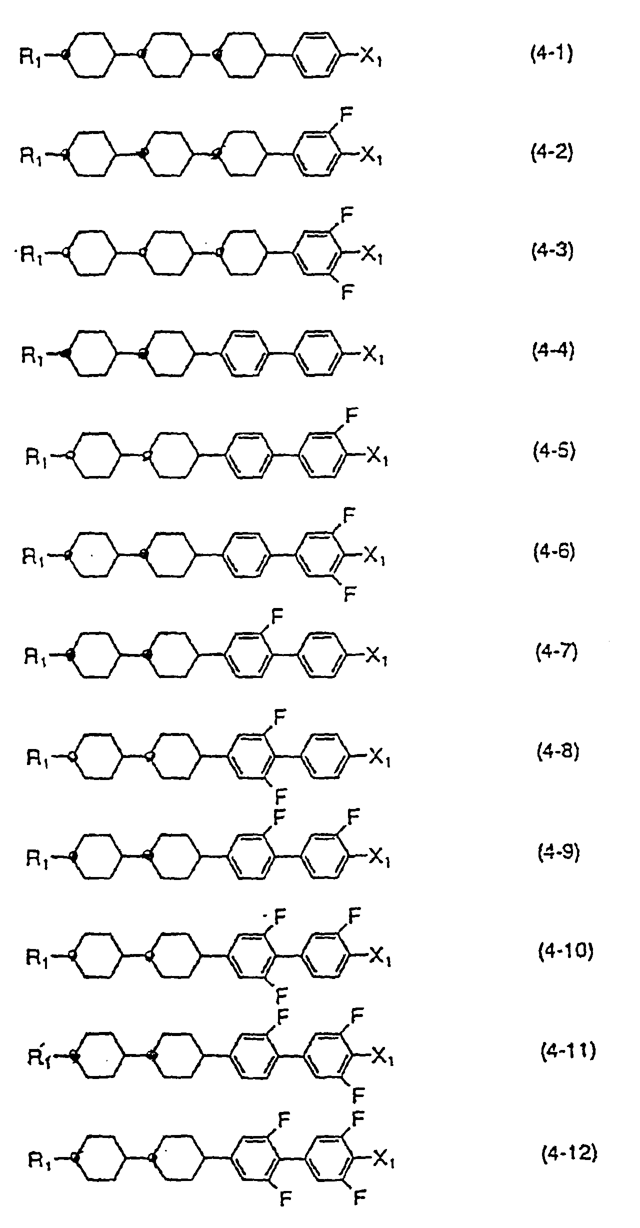 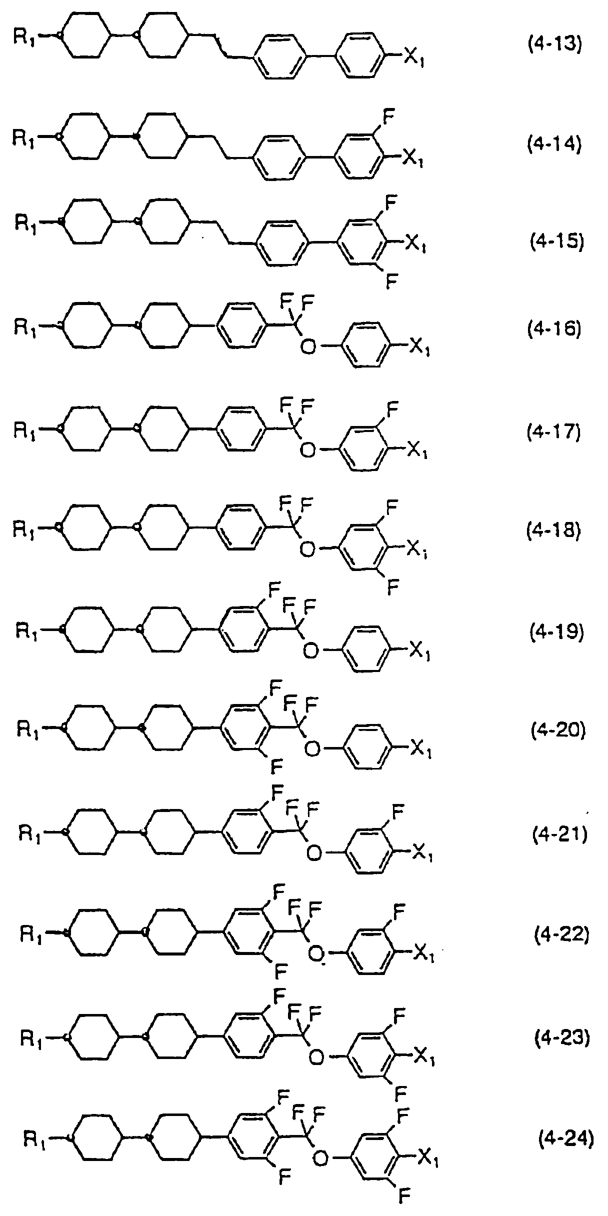 where, R₁ and X₁ have the same meanings as described above.

Compounds represented by general formulas (2) to (4) have positive anisotropic dielectric constants, excel in thermal and chemical stability, and are very useful for the preparation of liquid crystal compositions for TFT displays requiring high reliability; e.g., high voltage holding ratios and large resistivities.

In the preparation of liquid crystal compositions for TFT displays, the compounds represented by general formulas (2) to (4) can be used within the quantity range of 0.1 to 99.9% by weight, preferably 10 to 97% by weight, and more preferably 40 to 95% by weight. Compounds represented by general formulas (7) to (9) may further be contained for adjusting viscosity.

Although compounds represented by general formulas (2) to (4) can be used for preparing liquid crystal compositions for STN and TN displays, the total content of these compounds is preferably not more than 50% by weight.

Preferred examples of compounds represented by general formulas (5) and (6) used in the liquid crystal composition of the present invention are as follows: 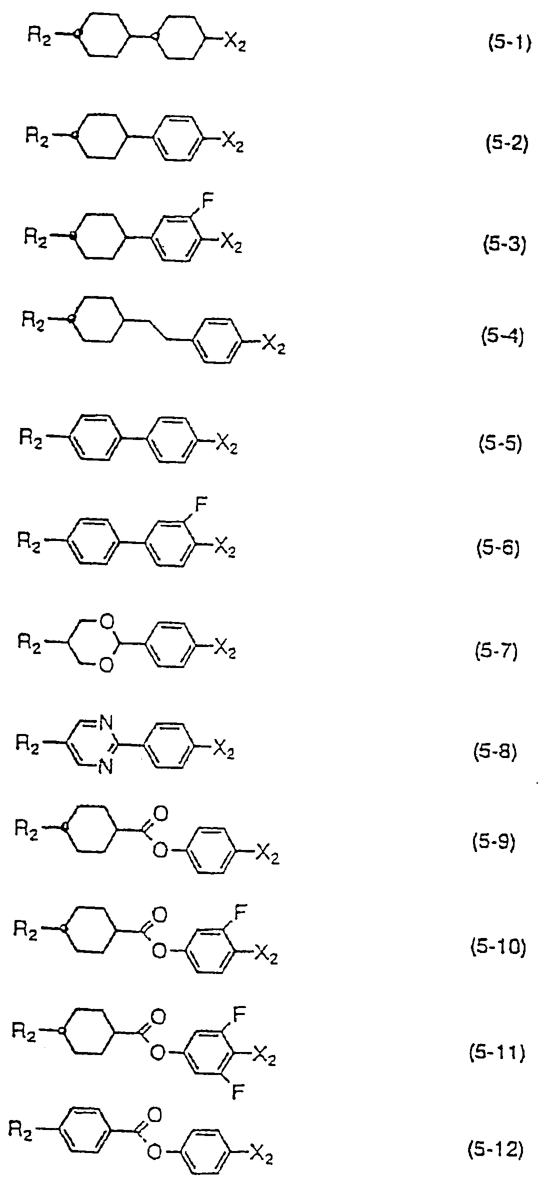 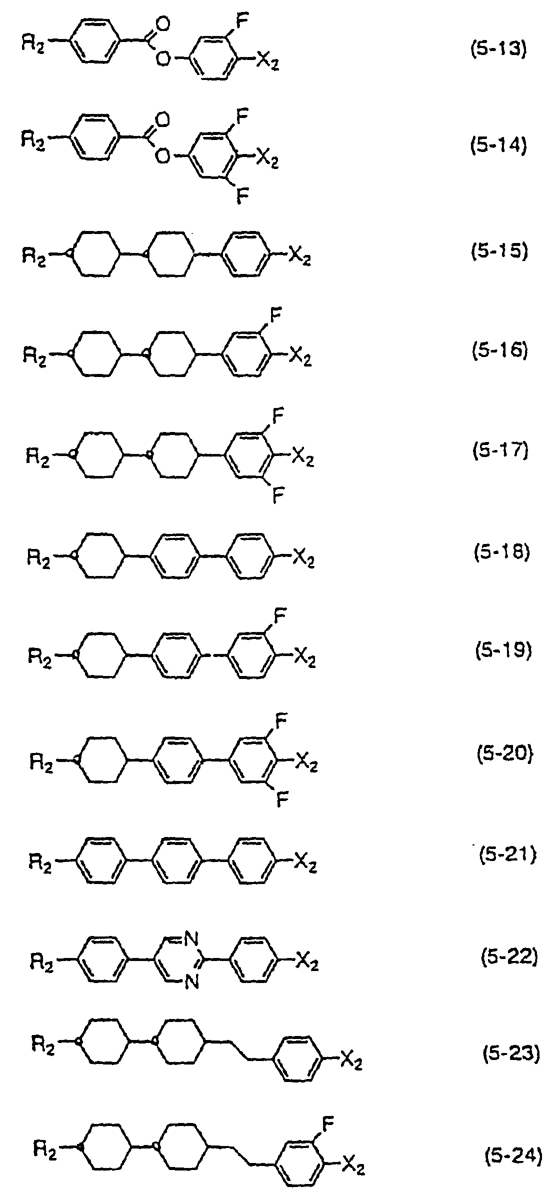 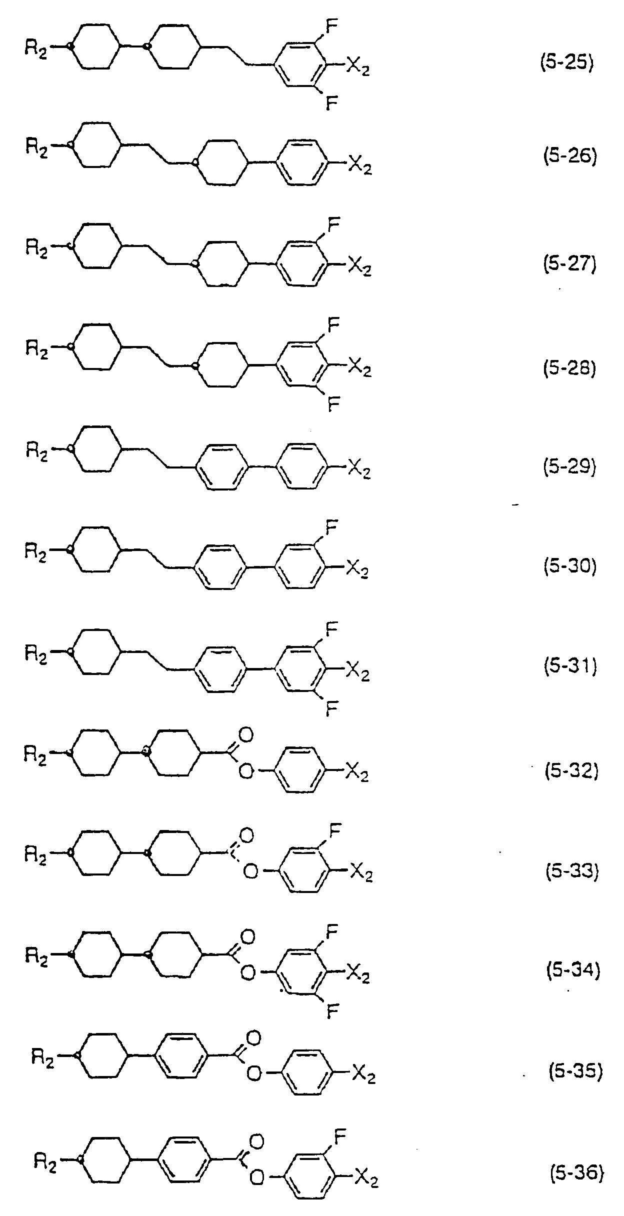 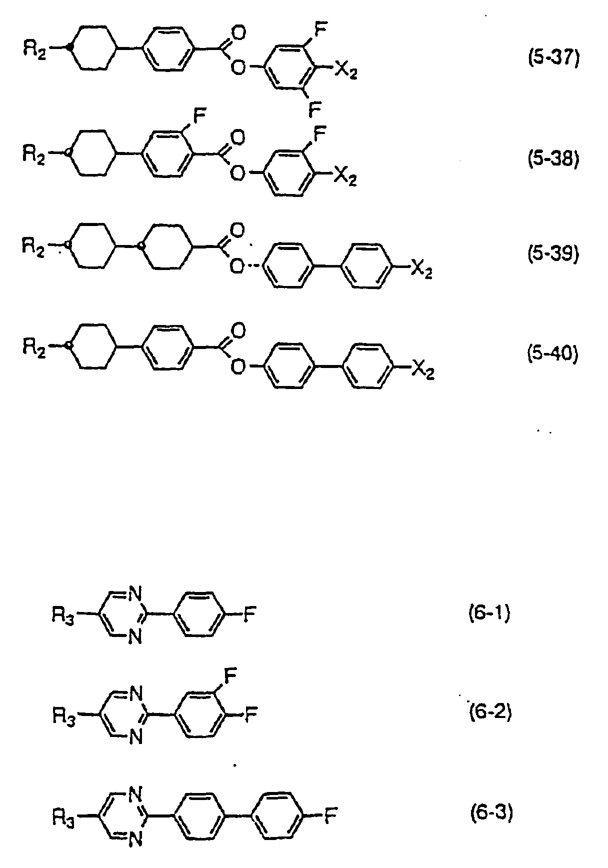 where, R₂, R₃, and X₂ have the same meanings as described above.

Compounds represented by general formulas (5) and (6) have large, positive anisotropic dielectric constants, and are used for lowering the threshold voltages of the liquid crystal composition. These compounds are also used for expanding the nematic range by adjusting anisotropic refractive indices or elevating transparency points. Furthermore, these compounds are also used for the improvement of steepness of the liquid crystal compositions for STN or TN displays.

Compounds represented by general formulas (5) to (6) are particularly useful for the preparation of liquid crystal compositions for STN or TN displays.

When the quantity of compounds represented by general formulas (5) to (6) used in a liquid crystal composition increases, the threshold voltage of the liquid crystal composition decreases, but its viscosity increases. Therefore, so long as the viscosity of the liquid crystal composition satisfies the requirements, use of a greater quantity of the compounds is advantageous, because the displays can be driven at a lower voltage. In the preparation of liquid crystal compositions for STN or TN displays, the compounds represented by general formulas (5) and (6) can be used within the quantity range of 0.1 to 99.9% by weight, preferably 10 to 97% by weight, and more preferably 40 to 95% by weight.

Preferred examples of compounds represented by general formulas (7) to (9) used in the liquid crystal composition of the present invention are as follows: 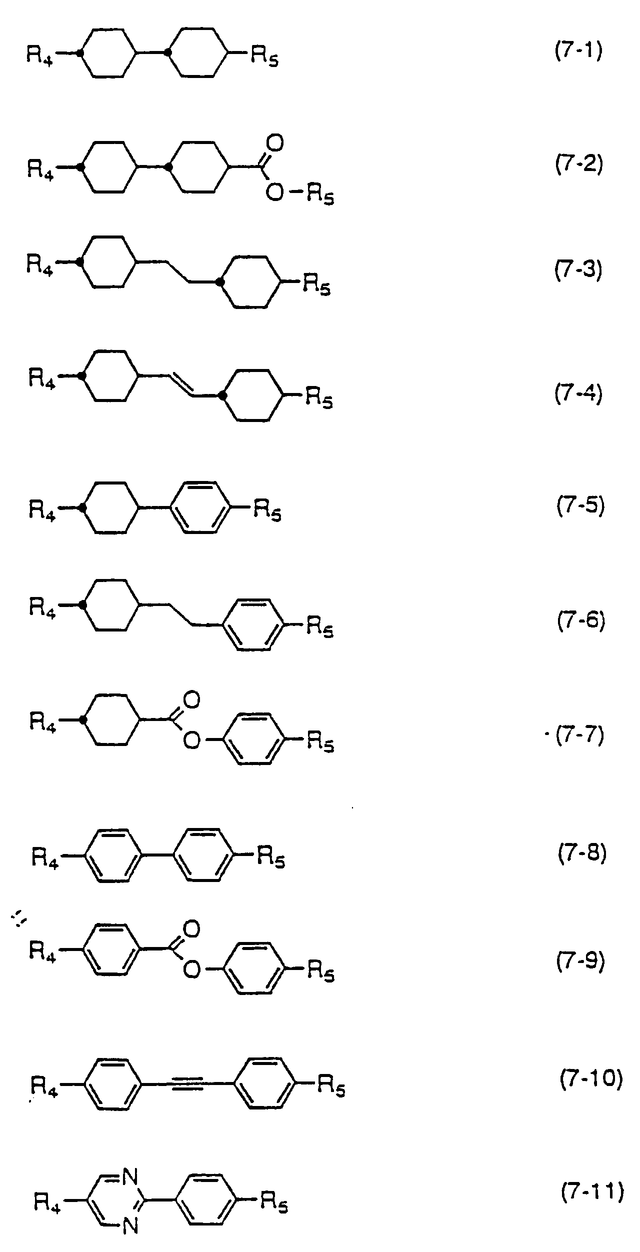 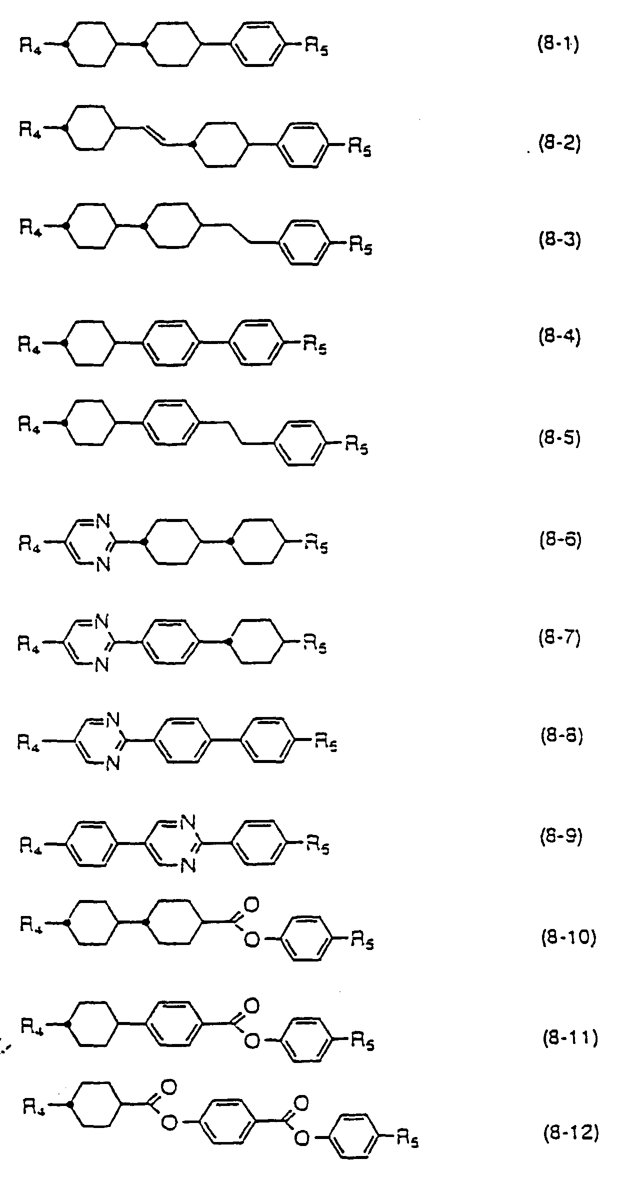 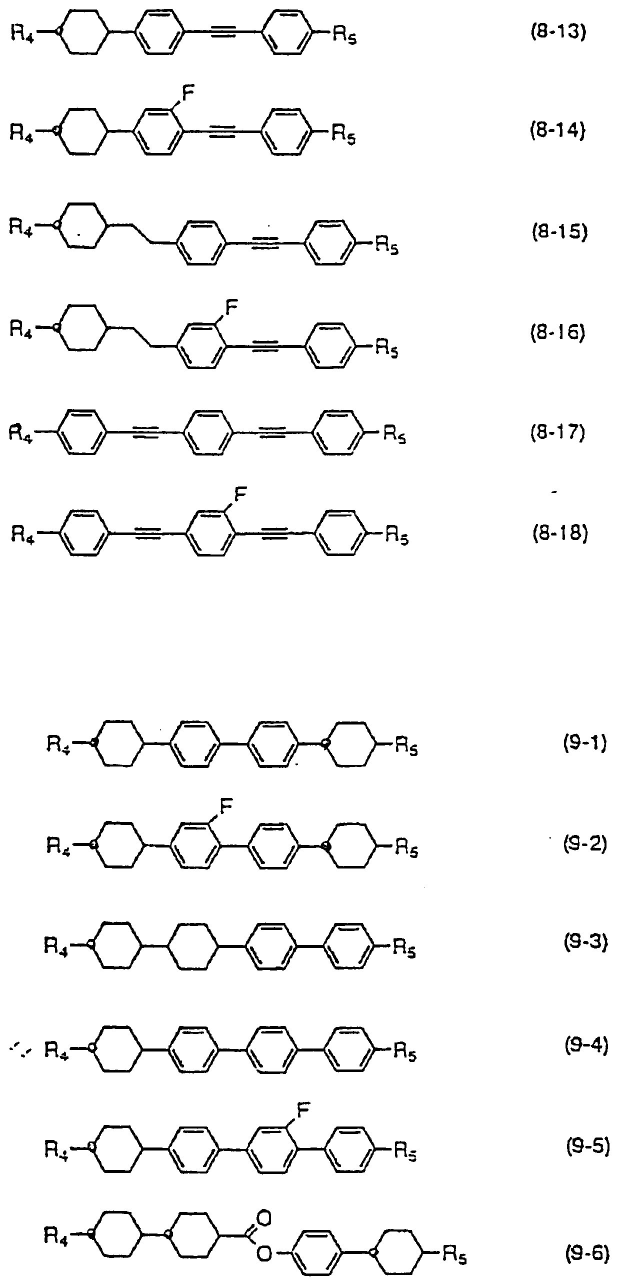 where, R₄ and R₅ have the same meanings as described above.

Compounds represented by general formulas (7) to (9) have small absolute values of anisotropic dielectric constants, and are nearly neutral. Compounds represented by general formula (7) are mainly used for adjusting viscosity or anisotropic refractive indices. Compounds represented by general formulas (8) and (9) are used for expanding the nematic range; for example, thorugh elevating clearing points, or for adjusting anisotropic reflection indices.

When the quantity of compounds represented by general formulas (7) to (9) used in a liquid crystal composition increases, the threshold voltage of the liquid crystal composition increases, but its viscosity decreases. Therefore, so long as the threshold voltage of the liquid crystal composition satisfies the above requirements, use of a larger quantity of the compounds is preferred. In the preparation of liquid crystal compositions for TFT displays, the total quantity of compounds represented by general formulas (7) to (9) is preferably 40% by weight or less, more preferably 35% by weight or less. In the preparation of liquid crystal compositions for STN or TN displays, the total quantity of the compounds is preferably 70% by weight or less, more preferably 60% by weight or less.

In the present invention, an optically active compound is normally added for adjusting required twist angle by inducing the spiral structure of the liquid crystal composition, and preventing reverse twist, except in particular cases such as liquid crystal compositions for OCB (optically compensated birefringence) mode. As optically active compounds of the present invention, optically active compounds well known to the art may be used, and preferred examples include the following optically active compounds.

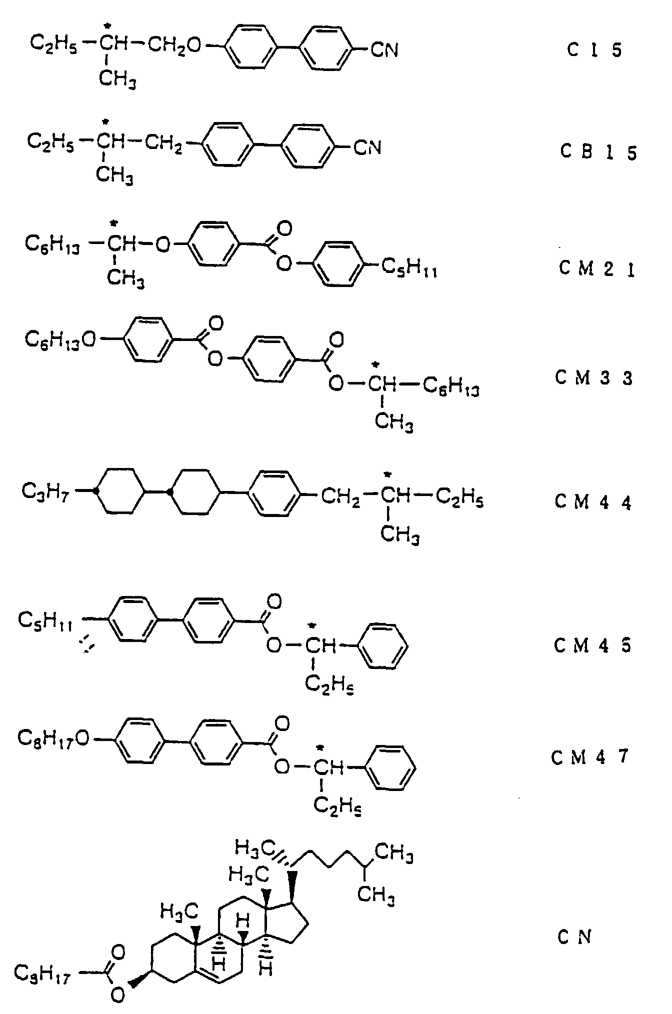

Normally, these optically active compounds are added to the liquid crystal composition of the present invention for adjusting the pitch of twist. The pitch of twist is preferably adjusted within a range of 40 to 200 µm for liquid crystal compositions for TFT and TN mode displays, and within a range of 6 to 20 µm for liquid crystal compositions for STN mode displays. For bistable TN mode displays, the pitch of twist is preferably adjusted within a range of 1.5 to 4 µm. Two or more optically active compounds may be added for adjusting the temperature dependence of the pitch.

The liquid crystal compositions of the present invention are prepared by commonly used techniques. Typically, various components are dissolved with each other at high temperature.

The liquid crystal compositions of the present invention can also be used as the liquid crystal compositions for guest-host (GH) mode displays by addition of dichromatic colorants such as merocyanine, styryl, azo, azomethyne, azoxy, quinophthalone, anthraquinone, and tetrazine dyes. The liquid crystal compositions of the present invention can also be used as the liquid crystal compositions for NCAPs produced by encapsulating nematic liquid crystals in micro capsules, or polymer dispersion-type liquid crystal display (PDLCD) elements represented by polymer network liquid crystal display (PNLCD) elements in which a three-dimensional matrix is formed in liquid crystals. In addition, such compositions can also be used as liquid crystal compositions for birefringence control (ECB) mode or dynamic scattering (DS) mode displays.

Examples of liquid crystal compositions containing the compounds of the present invention include the following. Compounds in the examples and in embodiments described below are represented by abbreviations according to the rules shown below. Compound numbers are the same as those shown in embodiments described below. In examples and embodiments, "percentage" means "percentage by weight" unless otherwise specified.

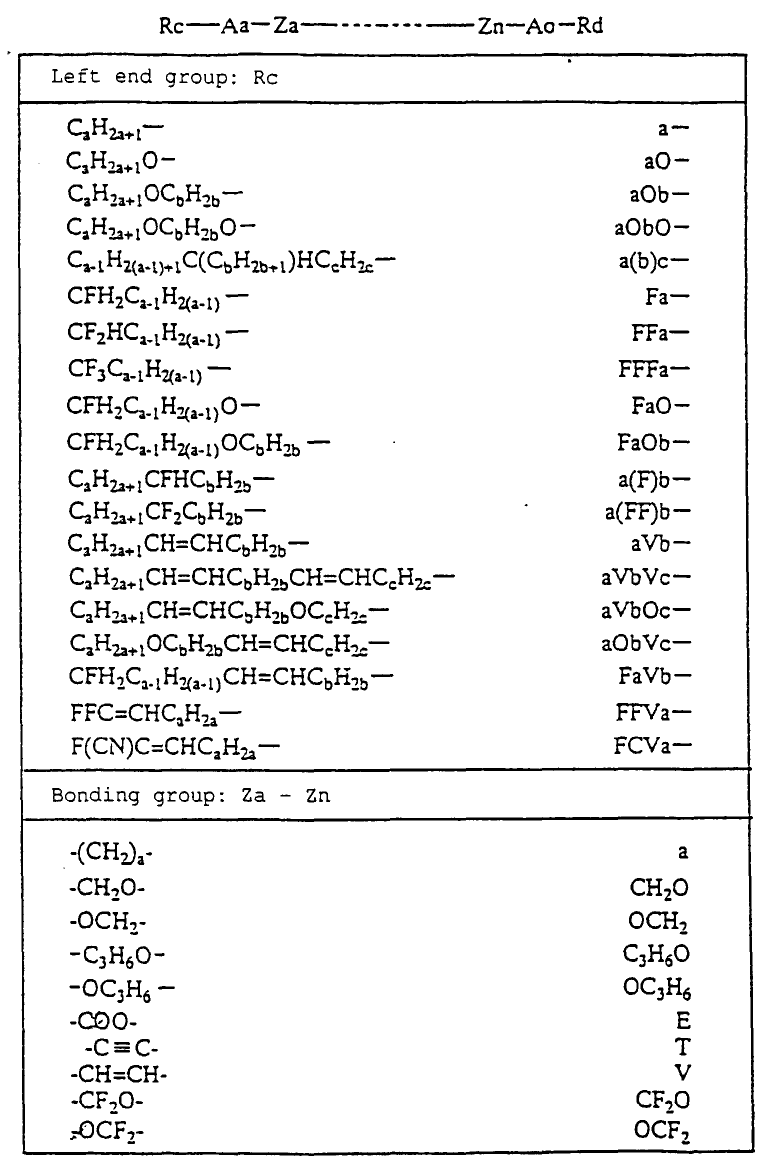

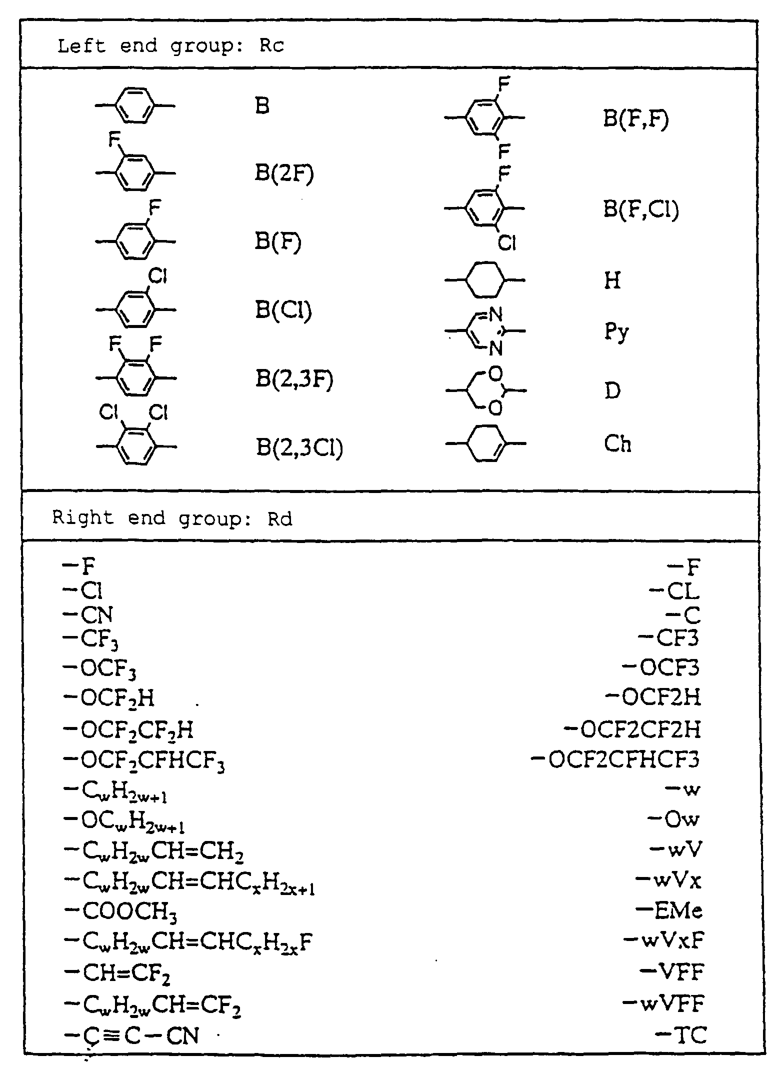

| Composition example 1 | |
|---|---|
| 3-B2B(F)B(F,F)-F (Compound No. 13) | 10.0% |
| 1V2-BEB(F,F)-C | 5.0% |
| 3-HB-C | 25.0% |
| 1-BTB-3 | 5.0% |
| 3-HH-4 | 11.0% |
| 3-HHB-1 | 11.0% |
| 3-HHB-3 | 9.0% |
| 3-H2BTB-2 | 4.0% |
| 3-H2BTB-3 | 4.0% |
| 3-H2BTB-4 | 4.0% |
| 3-HB(F)TB-2 | 6.0% |
| 3-HB(F)TB-3 | 6.0% |

| Composition example 2 | |
|---|---|
| 3-B(F)2B(F)B(F,F)-F (Compound No. 1) | 6.0% |
| 3-B(F)2B(F,F)B(F)-F (Compound No. 68) | 6.0% |
| V2-HB-C | 12.0% |
| 1V2-HB-C | 12.0% |
| 3-HB-C | 12.0% |
| 3-HB(F)-C | 5.0% |
| 2-BTB-1 | 2.0% |
| 3-HH-4 | 8.0% |
| 3-HH-VFF | 6.0% |
| 2-HHB-C | 3.0% |
| 3-HHB-C | 6.0% |
| 3-HB(F)TB-2 | 8.0% |
| 3-H2BTB-2 | 5.0% |
| 3-H2BTB-3 | 5.0% |
| 3-H2BTB-4 | 4.0% |

| Composition example 3 | |
|---|---|
| 3-B(F)2B(F,F)B(F)-CL (Compound No. 71) | 5.0% |
| 3-BB(F,F)2B(F)-CL (Compound No. 115) | 2.0% |
| 3O1-BEB(F)-C | 13.0% |
| 4O1-BEB(F)-C | 13.0% |
| 5O1-BEB(F)-C | 13.0% |
| 2-HHB(F)-C | 15.0% |
| 3-HHB(F)-C | 15.0% |
| 3-HB(F)TB-2 | 4.0% |
| 3-HB(F)TB-3 | 4.0% |
| 3-HB(F)TB-4 | 4.0% |
| 3-HHB-1 | 8.0% |
| 3-HHB-O1 | 4.0% |

| Composition example 4 | |
|---|---|
| 3-B(F)2B(F)B(F,F)-F (Compound No. 1) | 5.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 3-PyB-2 | 2.0% |
| 4-PyB-2 | 2.0% |
| 6-PyB-O5 | 3.0% |
| 6-PyB-O6 | 3.0% |
| 6-PyB-O7 | 3.0% |
| 6-PyB-O8 | 3.0% |
| 3-PyBB-F | 6.0% |
| 4-PyBB-F | 6.0% |
| 5-PyBB-F | 6.0% |
| 3-HHB-1 | 6.0% |
| 3-HHB-3 | 8.0% |
| 2-H2BTB-2 | 4.0% |
| 2-H2BTB-3 | 4.0% |
| 2-H2BTB-4 | 5.0% |
| 3-H2BTB-2 | 5.0% |
| 3-H2BTB-3 | 5.0% |

| Composition example 5 | |
|---|---|
| 3-B(F)2B(F,F)B(F)-F (Compound No. 68) | 10.0% |
| 3-DB-C | 10.0% |
| 2-BEB-C | 12.0% |
| 3-BEB-C | 4.0% |
| 3-PyB(F)-F | 6.0% |
| 3-HEB-O4 | 8.0% |
| 4-HEB-O2 | 6.0% |
| 5-HEB-O1 | 6.0% |
| 3-HEB-O2 | 5.0% |
| 5-HEB-O2 | 4.0% |
| 5-HEB-5 | 5.0% |
| 4-HEB-5 | 5.0% |
| 1O-BEB-2 | 4.0% |
| 3-HHB-1 | 6.0% |
| 3-HHEBB-C | 3.0% |
| 3-HBEBB-C | 3.0% |
| 5-HBEBB-C | 3.0% |

| Composition example 6 | |
|---|---|
| 3-BB(F,F)2B(F)-CL (Compound No. 115) | 3.0% |
| 3-B2B(F)B(F,F)-F (Compound No. 13) | 7.0% |
| 3-HB-C | 18.0% |
| 1O1-HB-C | 10.0% |
| 3-HB(F)-C | 10.0% |
| 2-PyB-2 | 2.0% |
| 3-PyB-2 | 2.0% |
| 4-PyB-2 | 2.0% |
| 2-BTB-O1 | 7.0% |
| 3-HHB-1 | 7.0% |
| 3-HHB-F | 4.0% |
| 3-HHB-O1 | 4.0% |
| 3-HB-O2 | 8.0% |
| 3-H2BTB-2 | 3.0% |
| 3-H2BTB-3 | 3.0% |
| 2-PyBH-3 | 4.0% |
| 3-PyBH-3 | 3.0% |
| 3-PyBB-2 | 3.0% |

| Composition example 7 | |
|---|---|
| 3-B(F)2BB(F)-CF₂H (Compound No. 11) | 3.0% |
| 2O1-BEB(F)-C | 5.0% |
| 3O1-BEB(F)-C | 9.0% |
| 5O1-BEB(F)-C | 4.0% |
| 1V2-BEB(F,F)-C | 10.0% |
| 3-HH-EMe | 10.0% |
| 3-HB-O2 | 18.0% |
| 7-HEB-F | 2.0% |
| 3-HHEB-F | 2.0% |
| 5-HHEB-F | 2.0% |
| 3-HBEB-F | 4.0% |
| 2O1-HBEB(F)-C | 2.0% |
| 3-HB(F)EB(F)-C | 2.0% |
| 3-HBEB(F,F)-C | 2.0% |
| 3-HHB-F | 4.0% |
| 3-HHB-O1 | 4.0% |
| 3-HHB-3 | 13.0% |
| 3-HEBEB-F | 2.0% |
| 3-HEBEB-1 | 2.0% |

| Composition example 8 | |
|---|---|
| 3-B2B(F)B(F,F)-F (Compound No. 13) | 2.0% |
| 3-B(F)2BB(F)-CF₂H (Compound No. 11) | 2.0% |
| 5-BEB(F)-C | 5.0% |
| V-HB-C | 11.0% |
| 5-PyB-C | 6.0% |
| 4-BB-3 | 7.0% |
| 3-HH-2V | 10.0% |
| 5-HH-V | 11.0% |
| V-HHB-1 | 7.0% |
| V2-HHB-1 | 15.0% |
| 3-HHB-1 | 9.0% |
| 1V2-HBB-2 | 10.0% |
| 3-HHEBH-3 | 5.0% |

| Composition example 9 | |
|---|---|
| 3-B(F)B(F)2B(F,F)-OCF₂H (Compound No. 159) | 2.0% |
| 3-B(F,F)B(F)2B(F,F)-CFH₂ (Compound No. 193) | 2.0% |
| 2O1-BEB(F)-C | 5.0% |
| 3O1-BEB(F)-C | 12.0% |
| 5O1-BEB(F)-C | 4.0% |
| 1V2-BEB(F,F)-C | 16.0% |
| 3-HB-O2 | 10.0% |
| 3-HH-4 | 3.0% |
| 3-HHB-F | 3.0% |
| 3-HHB-1 | 8.0% |
| 3-HHB-O1 | 4.0% |
| 3-HBEB-F | 4.0% |
| 3-HHEB-F | 7.0% |
| 5-HHEB-F | 7.0% |
| 3-H2BTB-2 | 4.0% |
| 3-H2BTB-3 | 4.0% |
| 3-HB(F)TB-2 | 5.0% |

| Composition example 10 | |
|---|---|
| 3-B2B(F)B(F,F)-F (Compound No. 13) | 2.0% |
| 3-B(F)2B(F)B(F,F)-F (Compound No. 1) | 2.0% |
| 3-B(F)2B(F,F)B(F)-F (Compound No. 68) | 2.0% |
| 3-BB(F,F)2B(F)-CL (Compound No. 115) | 2.0% |
| 2-BEB-C | 12.0% |
| 3-BEB-C | 4.0% |
| 4-BEB-C | 6.0% |
| 3-HB-C | 20.0% |
| 3-HEB-O4 | 12.0% |
| 4-HEB-O2 | 8.0% |
| 5-HEB-O1 | 8.0% |
| 3-HEB-O2 | 6.0% |
| 5-HEB-O2 | 5.0% |
| 3-HHB-1 | 7.0% |
| 3-HHB-O1 | 4.0% |

| Composition example 11 | |
|---|---|
| 3-B2B(F)B(F,F)-F (Compound No. 13) | 2.0% |
| 3-BB(F,F)2B(F)-CL (Compound No. 115) | 2.0% |
| 3-B(F)B(F)2B(F,F)-OCF₂H (Compound No. 159) | 2.0% |
| 2-BEB-C | 10.0% |
| 5-BB-C | 12.0% |
| 7-BB-C | 7.0% |
| 1-BTB-3 | 7.0% |
| 2-BTB-1 | 4.0% |
| 1O-BEB-2 | 10.0% |
| 1O-BEB-5 | 12.0% |
| 2-HHB-1 | 4.0% |
| 2-HHB-F | 4.0% |
| 3-HHB-1 | 7.0% |
| 3-HHB-O1 | 4.0% |
| 3-HHB-3 | 13.0% |

| Composition example 12 | |
|---|---|
| 3-B2B(F)B(F,F)-F (Compound No. 13) | 3.0% |
| 3-B(F)2B(F)B(F,F)-F (Compound No. 1) | 2.0% |
| 3-B(F)2B(F,F)B(F)-F (Compound No. 68) | 2.0% |
| 2-HHB(F)-F | 17.0% |
| 3-HHB(F)-F | 17.0% |
| 5-HHB(F)-F | 16.0% |
| 2-H2HB(F)-F | 10.0% |
| 3-H2HB(F)-F | 5.0% |
| 5-H2HB(F)-F | 10.0% |
| 2-HBB(F)-F | 6.0% |
| 3-HBB(F)-F | 6.0% |
| 5-HBB(F)-F | 6.0% |

| Composition example 13 | |
|---|---|
| 3-B2B(F)B(F,F)-F (Compound No. 13) | 5.0% |
| 3-B(F)2B(F,F)B(F)-CL (Compound No. 71) | 2.0% |
| 7-HB(F)-F | 5.0% |
| 5-H2B(F)-F | 5.0% |
| 3-HB-O2 | 10.0% |
| 3-HH-4 | 5.0% |
| 2-HHB(F)-F | 8.0% |
| 3-HHB(F)-F | 10.0% |
| 5-HHB(F)-F | 5.0% |
| 3-H2HB(F)-F | 5.0% |
| 2-HBB(F)-F | 3.0% |
| 3-HBB(F)-F | 3.0% |
| 5-HBB(F)-F | 6.0% |
| 2-H2BB(F)-F | 5.0% |
| 3-H2BB(F)-F | 6.0% |
| 3-HHB-1 | 8.0% |
| 3-HHB-O1 | 5.0% |
| 3-HHB-3 | 4.0% |

| Composition example 14 | |
|---|---|
| 3-B2B(F)B(F,F)-F (Compound No. 13) | 5.0% |
| 7-HB(F,F)-F | 3.0% |
| 3-HB-O2 | 7.0% |
| 2-HHB(F)-F | 10.0% |
| 3-HHB(F)-F | 10.0% |
| 5-HHB(F)-F | 10.0% |
| 2-HBB(F)-F | 9.0% |
| 3-HBB(F)-F | 9.0% |
| 5-HBB(F)-F | 16.0% |
| 2-HBB-F | 4.0% |
| 3-HBB-F | 4.0% |
| 5-HBB-F | 3.0% |
| 3-HBB(F,F)-F | 5.0% |
| 5-HBB(F,F)-F | 5.0% |

| Composition example 15 | |
|---|---|
| 3-B(F)2B(F)B(F,F)-F (Compound No. 1) | 7.0% |
| 7-HB(F,F)-F | 4.0% |
| 3-H2HB(F,F)-F | 5.0% |
| 4-H2HB(F,F)-F | 10.0% |
| 5-H2HB(F,F)-F | 10.0% |
| 3-HHB(F,F)-F | 10.0% |
| 4-HHB(F,F)-F | 5.0% |
| 3-HH2B(F,F)-F | 15.0% |
| 5-HH2B(F,F)-F | 10.0% |
| 3-HBB(F,F)-F | 12.0% |
| 5-HBB(F,F)-F | 12.0% |

| Composition example 16 | |
|---|---|
| 3-B(F)2B(F,F)B(F)-F (Compound No. 68) | 5.0% |
| 7-HB(F,F)-F | 5.0% |
| 3-H2HB(F,F)-F | 12.0% |
| 4-H2HB(F,F)-F | 10.0% |
| 3-HHB(F,F)-F | 10.0% |
| 4-HHB(F,F)-F | 5.0% |
| 3-HBB(F,F)-F | 5.0% |
| 3-HHEB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |
| 5-HBEB(F,F)-F | 3.0% |
| 3-HDB(F,F)-F | 15.0% |
| 3-HHBB(F,F)-F | 6.0% |

| Composition example 17 | |
|---|---|
| 3-B(F)2B(F,F)B(F)-CL (Compound No. 71) | 2.0% |
| 3-BB(F,F)2B(F)-CL (Compound No. 115) | 2.0% |
| 3-HB-CL | 10.0% |
| 5-HB-CL | 4.0% |
| 7-HB-CL | 4.0% |
| 1O1-HH-5 | 5.0% |
| 2-HBB(F)-F | 8.0% |
| 3-HBB(F)-F | 8.0% |
| 5-HBB(F)-F | 10.0% |
| 4-HHB-CL | 8.0% |
| 5-HHB-CL | 8.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |
| 3-HB(F)VB-3 | 4.0% |

| Composition example 18 | |
|---|---|
| 3-B2B(F)B(F,F)-F (Compound No. 13) | 2.0% |
| 3-B(F)2B(F)B(F,F)-F (Compound No. 1) | 2.0% |
| 3-B(F)2B(F,F)-B(F)-F (Compound No. 68) | 2.0% |
| 3-B(F)2B(F,F)-B(F)-CL (Compound No. 71) | 2.0% |
| 3-BB(F,F)2B(F)-CL (Compound No. 115) | 2.0% |
| 3-HHB(F,F)-F | 9.0% |
| 3-H2HB(F,F)-F | 8.0% |
| 4-H2HB(F,F)-F | 8.0% |
| 5-H2HB(F,F)-F | 8.0% |
| 3-HHB(F,F)-F | 21.0% |
| 5-HHB(F,F)-F | 10.0% |
| 3-H2BB(F,F)-F | 10.0% |
| 5-HHBB(F,F)-F | 3.0% |
| 3-HH2BB(F,F)-F | 3.0% |
| 5-HHEBB-F | 2.0% |
| 1O1-HBBH-4 | 4.0% |
| 1O1-HBBH-5 | 4.0% |

| Composition example 19 | |
|---|---|
| 3-B2B(F)B(F,F)-F (Compound No. 13) | 2.0% |
| 3-B(F)2BB(F)-CF₂H (Compound No. 11) | 2.0% |
| 3-B(F)B(F)2B(F,F)-OCF₂H (Compound No. 159) | 2.0% |
| 5-HB-F | 12.0% |
| 6-HB-F | 9.0% |
| 7-HB-F | 7.0% |
| 2-HHB-OCF₃ | 7.0% |
| 3-HHB-OCF₃ | 11.0% |
| 4-HHB-OCF₃ | 7.0% |
| 5-HHB-OCF₃ | 5.0% |
| 3-HH2B-OCF₃ | 4.0% |
| 5-HH2B-OCF₃ | 4.0% |
| 3-HHB(F,F)-OCF₃ | 5.0% |
| 3-HBB(F)-F | 10.0% |
| 5-HBB(F)-F | 4.0% |
| 3-HH2B(F)-F | 3.0% |
| 3-HB(F)BH-3 | 3.0% |
| 5-HBBH-3 | 3.0% |

| Composition example 20 | |
|---|---|
| 3-B(F)2B(F,F)B(F)-F (Compound No. 68) | 5.0% |
| 3-B(F)2B(F,F)B(F)-CL (Compound No. 71) | 5.0% |
| 5-H4HB(F,F)-F | 7.0% |
| 5-H4HB-OCF₃ | 10.0% |
| 3-H4HB(F,F)-CF₃ | 8.0% |
| 5-H4HB(F,F)-CF₃ | 5.0% |
| 3-HB-CL | 6.0% |
| 5-HB-CL | 4.0% |
| 2-H2BB(F)-F | 5.0% |
| 3-H2BB(F)-F | 10.0% |
| 5-HVHB(F,F)-F | 5.0% |
| 3-HHB-OCF₃ | 5.0% |
| 3-H2HB-OCF₃ | 5.0% |
| V-HHB(F)-F | 5.0% |
| 3-HChB(F)-F | 5.0% |
| 5-HHEB-OCF₃ | 2.0% |
| 3-HBEB(F,F)-F | 5.0% |
| 5-HH-V2F | 3.0% |

| Composition example 21 | |
|---|---|
| 3-B2B(F)B(F,F)-F (Compound No. 13) | 3.0% |
| 3-B(F)2B(F,F)B(F)-F (Compound No. 68) | 3.0% |
| 3-B(F,F)B(F)2B(F,F)-CFH₂ (Compound No. 193) | 3.0% |
| 2-HHB(F)-F | 2.0% |
| 3-HHB(F)-F | 2.0% |
| 5-HHB(F)-F | 2.0% |
| 2-HBB(F)-F | 6.0% |
| 3-HBB(F)-F | 6.0% |
| 5-HBB(F)-F | 10.0% |
| 2-H2BB(F)-F | 9.0% |
| 3-H2BB(F)-F | 9.0% |
| 3-HBB(F,F)-F | 25.0% |
| 5-HBB(F,F)-F | 10.0% |
| 1O1-HBBH-4 | 5.0% |
| 1O1-HBBH-5 | 5.0% |

### Best Mode for Carrying Out the Invention

The present invention will be described in further detail by reference to embodiments. In each embodiment, C represents a crystal, SA represents a smectic phase A, SB represents a smectic phase B, SX represents a smectic phase not determined, N represents a nematic phase, and Iso represents an isotropic phase. The unit for all transition temperatures is °C.

### Embodiment 1

### Synthesis of 4'-(2-(2-fluoro-4-propylphenyl)ethyl)-2'-fluoro-3,4,5-trifluorobiphenyl (compound (No. 1) represented by general formula (1), where R is C₃H₇, each of Y₁, Y₃, Y₅, Y₆, and X is F, Z₁ is -(CH₂)₂-, and Z₂ is a covalent bond) (Step 1) synthesis of (2-(2-fluoro-4-propylphenyl)ethyl)-3-fluoro-4-iodobenzene

Into a solution of 2.6 g (10.1 mmol) of (2-(2-fluoro-4-propylphenyl)ethyl)-3-fluorobenzene dissolved in 40 ml of tetrahydrofuran (THF), 11 ml (11.1 mmol) of sec-BuLi was added dropwise at a speed that maintains a temperature of -60°C or below, and after completion of addition, the solution was stirred for 2 hours at the same temperature. Then, a solution of 2.9 g (11.6 mmol) of iodine dissolved in 20 ml of THF was added dropwise at a speed that maintains a temperature of -60°C or below, and the solution was stirred for 1 hour at the same temperature.

After dropwise addition of 50 ml of 1N-HCl into the reaction solution, the solution was extracted with 50 ml of heptane. The organic layer obtained was washed three times with diluted NaHCO₃ solution and three times with water, and dried over anhydrous magnesium sulfate. The solvent was distilled off under a reduced pressure, and the residue was subjected to silica-gel-column chromatography to obtain 3.5 g of crude (2-(2-fluoro-4-propylphenyl)ethyl)-3-fluoro-4-iodobenzene (yield: 90.5%).

### (Step 2) Synthesis of 4'-(2-(2-fluoro-4-propylphenyl)ethyl)-2'-fluoro-3,4,5-trifluorobiphenyl

The mixture of 3.5 g (9.1 mmol) of (2-(2-fluoro-4-propylphenyl)ethyl)-3-fluoro-4-iodobenzene obtained in the previous step, 2.1 g (11.9 mmol) of dihydroxy(3,4,5-trifluorophenyl)borane, 2.5 g (18.3 mmol) of K₂CO₃, 0.3 g of 5% Pd-C, and 30 ml of a toluene/ethanol/water (1/1/1) mixed solvent was heated and refluxed for 12 hours. Then, after Pd-C was removed by filtration, the solution was extracted by 100 ml of toluene, and the obtained organic layer was washed three times with water and dried over anhydrous magnesium sulfate. The solvent was distilled off under reduced pressure, and the residue was subjected to silica-gel-column chromatography (eluent: heptane) to obtain 2.5 g of crude 4'-(2-(2-fluoro-4-propylphenyl)ethyl)-2'-fluoro-3,4,5-trifluorobiphenyl.

The product was recrystallized from ethanol to form 1.2 g of the desired compound (yield: 34.3%).

The transition temperature of this compound was: C 66.6-67.8 Iso

All spectrum data well supported its structure.
Mass spectrometry: 390(M⁺)
¹H-NMR (CDCl₃, TMS internal standard)
δ (ppm)
0.93 (t, 3H)
1.54-1.75 (m, 2H)
2.56 (t, 2H)
2.92 (s, 4H)
6.80-7.36 (m, 8H)

Examples of cases where the compounds of the present invention were used as the components of liquid crystal compositions will be shown below. In these examples, NI represents a nematic phase-isotropic transition temperature (°C), Δε represents an anisotropic dielectric constant value, Δn represents an anisotropic refractive index value, η represents viscosity (mPa·s), and Vth represents a threshold voltage (V).

η was measured at 20°C, and Δε, Δn, and Vth were measured at 25°C.

### Embodiment 2 (Use Example 1)

Liquid crystal composition (A) containing the following cyanophenyl cyclohexane liquid crystalline compounds:
- 4-(*trans*-4-propylcyclohexyl)benzonitrile: 24%
- 4-(*trans*-4-pentylcyclohexyl)benzonitrile: 36%
- 4-(*trans*-4-heptylcyclohexyl)benzonitrile: 25%
4-(*trans*-4-pentylcyclohexyl)-4'-cyanobiphenyl has the following properties:
NI: 71.7, Δε: 11.0, Δn: 0.137, η: 26.7, Vth: 1.78

The properties of liquid crystal composition (B) consisting of 85% composition (A) and 15% 4'-(2-(2-fluoro-4-propylphenyl)ethyl)-2'-fluoro-3,4,5-trifluorobiphenyl (compound No. 1) obtained in Embodiment 1 are as follows:
Δε: 12.4, Δn: 0.133, η: 30.2, Vth: 1.35

Although this liquid crystal composition (B) was stored in a freezer of a temperature of -20°C, neither appearance of a smectic phase nor deposition of crystals was observed even after 60 days.

### Embodiment 3 (Use Example 2)

The following compounds can be synthesized according to the method of Embodiment 1. Properties shown here were measured in the same manner as in Embodiment 2.
Compound No. 2: 1-B(F)2BB(F,F)-F
Compound No. 3: 2-B(F)2BB(F)-F
Compound No. 4: 3-B(F)2BB(F,F)-CL
Compound No. 5: 5-B(F)2BB(F)-CL
Compound No. 6: 3O-B(F)2BB(F,F)-OCF₃
Compound No. 7: 3-B(F)2BB(F)-OCF₃
Compound No. 8: 4-B(F)2BB(F,F)-CF₃
Compound No. 9: 5-B(F)2BB(F)-CF₃
Compound No. 10: 1O3O-B(F)2BB(F,F)-CF₂H
Compound No. 11: 3-B(F)2BB(F,F)-CF₂H
Compound No. 12: 2-B(F)2BB(F)-CFH₂
Compound No. 13: 3-B2B(F)B(F,F)-F
   Δε: 12.1, Δn: 0.133, η:30.5
Compound No. 14: 4-B2B(F)B(F,F)-CL
Compound No. 15: 5-B2B(F)B(F,F)-OCF₃
Compound No. 16: 6-B2B(F)B(F,F)-OCF₂H
Compound No. 17: 7-B2B(F)B(F,F)-CF₃
Compound No. 18: 1O-B2B(F)B(F,F)-CF₂H
Compound No. 19: 20-B2B(F)B(F,F)-CFH₂
Compound No. 20: 4-B(F,F)2BB(F,F)-F
Compound No. 21: 5-B(F,F)2BB(F)-F
Compound No. 22: 3O1-B(F,F)2BB(F,F)-CL
Compound No. 23: 3O1O-B(F,F)2BB(F)-CL
Compound No. 24: 3-B(F,F)2BB-CL
Compound No. 25: 3-B(F,F)2BB(F,F)-OCF₃
Compound No. 26: 4-B(F,F)2BB(F)-OCF₃
Compound No. 27: 4-B(F,F)2BB-OCF₃
Compound No. 28: 5-B(F,F)2BB(F,F)-OCF₂H
Compound No. 29: 5-B(F,F)2BB(F)-OCF₂H
Compound No. 30: 15O-B(F,F)2BB-OCF₂H
Compound No. 31: 2-B(F,F)2BB(F,F)-CF₃
Compound No. 32: 3-B(F,F)2BB(F)-CF₃
Compound No. 33: 2-B(F,F)2BB(F)-CF₂H
Compound No. 34: 3-B(F,F)2BB-CF₂H
Compound No. 35: 4-B(F,F)2BB(F,F)-CFH₂
Compound No. 36: 5-B(F)2B(F)B(F,F)-F
Compound No. 37: 7-B(F)2B(F)B-F
Compound No. 38: 8-B(F)2B(F)B(F,F)-CL
Compound No. 39: 5-B(F)2B(F)B-CL
Compound No. 40: 4-B(F)2B(F)B(F,F)-OCF₃
Compound No. 41: 3-B(F)2B(F)B-OCF₃
Compound No. 42: 1O5-B(F)2B(F)B(F,F)-OCF₂H
Compound No. 43: 5-B(F)2B(F)B-OCF₂H
Compound No. 44: 3-B(F)2B(F)B(F,F)-CF₃
Compound No. 45: 4-B(F)2B(F)B-CF₃
Compound No. 46: 2-B(F)2B(F)B(F,F)-CF₂H
Compound No. 47: 5-B(F)2B(F)B-CF₂H
Compound No. 48: 3-B(F)2B(F)B(F,F)-CFH₂
Compound No. 49: 3-B2B(F,F) B(F,F)-OCF₃
   Δε: 13.6, Δn: 0.127, η:27.0
Compound No. 50: 4-B2B(F,F) B(F)-OCF₃
Compound No. 51: 3-B2B(F,F) B(F,F)-OCF₂H
Compound No. 52: 3-B2B(F,F) B(F)-OCF₂H
Compound No. 53: 5-B2B(F,F) B(F,F)-CF₃
Compound No. 54: 2-B2B(F,F) B(F,F)-CF₂H
Compound No. 55: 2-B(F,F)2B(F)B(F)-F
Compound No. 56: 4-B(F,F)2B(F)B-F
Compound No. 57: 6-B(F,F)2B(F)B(F,F)-CL
Compound No. 58: 5-B(F,F)2B(F)B(F)-CL
Compound No. 59: 3-B(F,F)2B(F)B(F)-OCF₃
Compound No. 60: 1-B(F,F)2B(F)B-OCF₃
Compound No. 61: 4O-B(F,F)2B(F)B(F)-OCF₂H
Compound No. 62: 9O5-B(F,F)2B(F)B-OCF₂H
Compound No. 63: 3-B(F,F)2B(F)B(F)-CF₃
   Δε: 14.2, Δn: 0.127, η:28.9
Compound No. 64: 5-B(F,F)2B(F)B-CF₃
Compound No. 65: 2-B(F,F)2B(F)B(F)-CF₂H
Compound No. 66: 4-B(F,F)2B(F)B-CF₂H
Compound No. 67: 3-B(F,F)2B(F)B(F,F)-CFH₂
Compound No. 68: 3-B(F)2B(F,F)B(F)-F
   Δε: 12.4, Δn: 0.132, η: 31.6
Compound No. 69: 5-B(F)2B(F,F)B-F
Compound No. 70: 5-B(F)2B(F,F)B(F,F)-CL
Compound No. 71: 3-B(F)2B(F,F)B(F)-CL
   Δε: 12.5, Δn: 0.139, η: 32.6
Compound No. 72: 4-B(F)2B(F,F)B-CL
Compound No. 73: 2-B(F)2B(F,F)B(F,F)-OCF₃
Compound No. 74: 1-B(F)2B(F,F)B(F)-OCF₃
Compound No. 75: 6-B(F)2B(F,F)B-OCF₃
Compound No. 76: 3-B(F)2B(F,F)B(F,F)-OCF₂H
Compound No. 77: 3-B(F)2B(F,F)B(F)-OCF₂H
Compound No. 78: 7-B(F)2B(F,F)B-OCF₂H
Compound No. 79: 4-B(F)2B(F,F)B(F,F)-CF₃
Compound No. 80: 5-B(F)2B(F,F)B(F)-CF₃
Compound No. 81: 5-B(F)2B(F,F)B-CF₃
Compound No. 82: 4-B(F)2B(F,F)B(F,F)-CF₂H
Compound No. 83: 3-B(F)2B(F,F)B(F)-CF₂H
Compound No. 84: 3-B(F)2B(F,F)B-CF₂H
Compound No. 85: 2-B(F)2B(F,F)B(F)-CFH₂
Compound No. 86: 5-B(F,F)2B(F,F)B(F)-F
Compound No. 87: 5O1-B(F,F)2B(F,F)B-F
Compound No. 88: 3-B(F,F)2B(F,F)B(F,F)-CL
Compound No. 89: 4-B(F,F)2B(F,F)B(F)-CL
Compound No. 90: 4-B(F,F)2B(F,F)B(F)-OCF₃
Compound No. 91: 5-B(F,F)2B(F,F)B-OCF₃
Compound No. 92: 3-B(F,F)2B(F,F)B(F)-OCF₂H
Compound No. 93: 2-B(F,F)2B(F,F)B-OCF₂H
Compound No. 94: 2-B(F,F)2B(F,F)B(F)-CF₃
Compound No. 95: 5-B(F,F)2B(F,F)B(F)-CF₂H
Compound No. 96: 5-B(F,F)2B(F,F)B(F,F)-CF₂H
Compound No. 97: 3-B(F)4BB(F,F)-F
Compound No. 98: 4-B(F)4BB(F)-OCF₃
Compound No. 99: 5-B4B(F)B(F)-CL
Compound No. 100: 4-B4B(F)B(F,F)-CF₃
Compound No. 101: 5-B(F,F)4BB(F)-OCF₃
Compound No. 102: 3-B(F,F)4BB(F,F)-OCF₂H
Compound No. 103: 4-B(F)4B(F)B(F)-F
Compound No. 104: 2-B(F)4B(F)B(F)-CF₃
Compound No. 105: 3-B4B(F,F)B(F,F)-F
Compound No. 106: 5-B4B(F,F)B(F)-CF₃
Compound No. 107: 2-B(F,F)4B(F)B(F,F)-F
Compound No. 108: 4-B(F,F)4B(F)B(F)-CF₂H
Compound No. 109: 1-B(F)4B(F,F)B(F,F)-F
Compound No. 110: 7-B(F)4B(F,F)B(F)-OCF₃
Compound No. 111: 6-B(F,F)4B(F,F)B(F,F)-OCF₃
Compound No. 112: 3-B(F,F)4B(F,F)B(F)-CF₃
Compound No. 113: 4-B(F)2B(F)2B(F,F)-OCF₃
Compound No. 114: 5-B(F)2B(F,F)2B(F,F)-F

### Embodiment 4

### Synthesis of 4'-propyl-3,5-difluoro-4-(2-(3-fluoro-4-chlorophenyl)ethyl)biphenyl (compound (No. 115) represented by general formula (1), where R is C₃H₇, each of Y₃, Y₄, and Y₅ is F, X is Cl, Z₁ is a covalent bond, and Z₂ is -(CH₂)₂-) (Step 1) Synthesis of 4'-propyl-3,5-difluoro-4-(2-(3-fluoro-4-chlorophenyl)-1-hydroxyethyl)biphenyl

Into a solution of 6.0 g (25.8 mmol) of 4'-propyl-3,5-difluorobiphenyl dissolved in 30 ml of THF, 27 ml (43.9 mmol) of n-BuLi was added dropwise at a speed that maintains a temperature of -60°C or below, and after completion of addition, the solution was stirred for 1 hour at the same temperature. Then, a solution of 7.2 g (45.2 mmol) of (3-fluoro-4-chlorophenyl)acetaldehyde dissolved in 35 ml of THF was added dropwise at a speed that maintains a temperature of -60°C or below, and the solution was stirred for 1 hour at the same temperature.

After dropwise addition 100 ml of 1N-HCl into the reaction solution, the solution was extracted with 150 ml of ethyl acetate. The obtained organic layer was washed three times with diluted NaHCO₃ solution and three times with water, and dried over anhydrous magnesium sulfate. The solvent was distilled off under reduced pressure, and the residue was subjected to silica-gel-column chromatography (eluent: heptane/ethyl acetate) to obtain 3.7 g of crude 4'-propyl-3,5-difluoro-4-(2-(3-fluoro-4-chlorophenyl)-1-hydroxyethyl)biphenyl (yield: 35.6%).

### (Step 2) Synthesis of 4'-propyl-3,5-difluoro-4-(2-(3-fluoro-4-chlorophenyl)ethyl)biphenyl

Into the solution of 3.7 g (9.1 mmol) of 4'-propyl-3,5-difluoro-4-(2-(3-fluoro-4-chlorophenyl)-1-hydroxyethyl)biphenyl obtained in the previous step, 18 ml of 1M-CH₂Cl₂ solution of titanium tetrachloride, and 20 ml of CH₂Cl₂, 1.6 g (13.7 mmol) of triethylsilane were added dropwise while cooling with ice, and stirred for 3 hours at room temperature. The solution was poured into 100 ml of ice water and extracted by 50 ml of CH₂Cl₂, and the obtained organic layer was washed twice with a diluted NaHCO₃ solution and three times with water, and dried over anhydrous magnesium sulfate. The solvent was distilled off under reduced pressure, and the residue was subjected to silica-gel-column chromatography (eluent: heptane) to obtain 2.8 g of crude 4'-propyl-3,5-difluoro-4-(2-(3-fluoro-4-chlorophenyl)ethyl)biphenyl. The product was recrystallized from a heptane/ethyl acetate (9/1) mixed solution to form 1.7 g of the desired compound (yield: 48.5%).

The transition temperature of this compound was: C 94.1-95.0 Iso

All spectrum data well support its structure.
Mass spectrometry: 388(M⁺)
¹H-NMR (CDCl₃, TMS internal standard)
δ (ppm)
0.96 (t, 3H)
1.53-1.79 (m, 2H)
2.63 (t, 2H)
2.91 (s, 4H)
6.86-7.50 (m, 9H)

### Embodiment 5 (Use Example 3)

The properties of liquid crystal composition (C) consisting of 85% composition (A) described above and 15% 4'-propyl-3,5-difluoro-4-(2-(3-fluoro-4-chlorophenyl)ethyl)biphenyl (compound No. 115) obtained in Embodiment 4 are as follows: NI: 64.7, Δε: 11.7, Δn: 0.142, η: 31.0, Vth: 1.60

Although this liquid crystal composition (C) was stored in a freezer of a temperature of -20°C, neither appearance of a smectic phase nor deposition of crystals was observed even after 60 days.

### Embodiment 6 (Use Example 4)

The following compounds can be synthesized according to the method of Embodiment 4. Properties shown here were measured in the same manner as in Embodiment 2.
Compound No. 116: 3-B(F)B2B(F)-OCF₃
Compound No. 117: 3-B(F)B2B(F)-OCF₂H
Compound No. 118: 3-B(F)B2B(F)-CF₂H
Compound No. 119: 3O-B(F)B2B(F,F)-CFH₂
Compound No. 120: 1-BB(F)2B(F,F)-F
Compound No. 121: 5-BB(F)2B-F
Compound No. 122: 2-BB(F)2B(F,F)-CL
Compound No. 123: 3-BB(F)2B-CL
Compound No. 124: 5-BB(F)2B(F,F)-OCF₃
Compound No. 125: 6-BB(F)2B-OCF₃
Compound No. 126: 4-BB(F)2B(F,F)-OCF₂H
Compound No. 127: 3-BB(F)2B-OCF₂H
Compound No. 128: 5-BB(F)2B(F,F)-CF₃
Compound No. 129: 4-BB(F)2B-CF₃
Compound No. 130: 3-BB(F)2B(F,F)-CF₂H
Compound No. 131: 4-BB(F)2B-CF₂H
Compound No. 132: 5-BB(F)2B(F)-CFH₂
Compound No. 133: 1-B(F,F)B2B(F,F)-F
Compound No. 134: 2-B(F,F)B2B(F)-F
Compound No. 135: 3-B(F,F)B2B-F
Compound No. 136: 4-B(F,F)B2B(F,F)-CL
Compound No. 137: 5-B(F,F)B2B(F)-CL
Compound No. 138: 7-B(F,F)B2B-CL
Compound No. 139: 2-B(F,F)B2B(F,F)-OCF₃
Compound No. 140: 4-B(F,F)B2B(F)-OCF₃
Compound No. 141: 16-B(F,F)B2B-OCF₃
Compound No. 142: 3-B(F,F)B2B(F,F)-OCF₂H
Compound No. 143: 2-B(F,F)B2B(F)-OCF₂H
Compound No. 144: 5-B(F,F)B2B-OCF₂H
Compound No. 145: 5-B(F,F)B2B(F,F)-CF₃
Compound No. 146: 4-B(F,F)B2B(F)-CF₃
Compound No. 147: 3-B(F,F)B2B-CF₃
Compound No. 148: 3-B(F,F)B2B(F,F)-CF₂H
Compound No. 149: 1O1-B(F,F)B2B(F)-CF₂H
Compound No. 150: 5-B(F,F)B2B-CF₂H
Compound No. 151: 6-B(F,F)B2B(F)-CFH₂
Compound No. 152: 2-B(F)B(F)2B(F,F)-F
Compound No. 153: 5-B(F)B(F)2B-F
Compound No. 154: 2-B(F)B(F)2B(F,F)-CL
Compound No. 155: 4-B(F)B(F)2B-CL
Compound No. 156: 3-B(F)B(F)2B(F,F)-OCF₃
Compound No. 157: 3-B(F)B(F)2B-OCF₃
Compound No. 158: 3-B(F)B(F)2B(F,F)-OCF₂H
Compound No. 159: 5-B(F)B(F)2B(F,F)-CF₃
Compound No. 160: 2-B(F)B(F)2B-CF₃
Compound No. 161: 5-B(F)B(F)2B(F,F)-CF₂H
Compound No. 162: 5-B(F)B(F)2B-CF₂H
Compound No. 163: 4-B(F)B(F)2B(F,F)-CFH₂
Compound No. 164: 5-BB(F,F)2B(F)-F
Compound No. 165: 6-BB(F,F)2B-F
Compound No. 166: 1-BB(F,F)2B(F)-CL
Compound No. 167: 7-BB(F,F)2B-CL
Compound No. 168: 4-BB(F,F)2B(F)-OCF₃
Compound No. 169: 2-BB(F,F)2B-OCF₃
Compound No. 170: 2-BB(F,F)2B(F)-OCF₂H
Compound No. 171: 5-BB(F,F)2B-OCF₂H
Compound No. 172: 3-BB(F,F)2B(F,F)-CF₃
Compound No. 173: 3-BB(F,F)2B(F)-CF₃
Compound No. 174: 5O1-BB(F,F)2B-CF₃
Compound No. 175: 4-BB(F,F)2B(F)-CF₂H
Compound No. 176: 6-BB(F,F)2B-CF₂H
Compound No. 177: 2-BB(F,F)2B(F,F)-CFH₂
Compound No. 178: 3-B(F,F)B(F)2B(F,F)-F
Compound No. 179: 3-B(F,F)B(F)2B(F)-F
Compound No. 180: 3-B(F,F)B(F)2B-F
Compound No. 181: 3-B(F,F)B(F)2B(F,F)-CL
   Δε: 12.6, Δn: 0.135, η: 28.6
Compound No. 182: 3-B(F,F)B(F)2B(F)-CL
Compound No. 183: 3-B(F,F)B(F)2B-CL
Compound No. 184: 3-B(F,F)B(F)2B(F,F)-OCF₃
Compound No. 185: 3-B(F,F)B(F)2B(F)-OCF₃
Compound No. 186: 3-B(F,F)B(F)2B-OCF₃
Compound No. 187: 5-B(F,F)B(F)2B(F,F)-OCF₂H
Compound No. 188: 5-B(F,F)B(F)2B(F)-OCF₂H
Compound No. 189: 5-B(F,F)B(F)2B-OCF₂H
Compound No. 190: 3-B(F,F)B(F)2B(F,F)-CF₃
   Δε: 14.9, Δn: 0.124, η: 27.4
Compound No. 191: 5-B(F,F)B(F)2B(F)-CF₃
Compound No. 192: 5-B(F,F)B(F)2B-CF₃
Compound No. 193: 3-B(F,F)B(F)2B(F,F)-CFH₂
Compound No. 194: 5-B(F,F)B(F)2B(F)-CF₂H
Compound No. 195: 5-B(F,F)B(F)2B-CF₂H
Compound No. 196: 5-B(F,F)B(F)2B(F)-CFH₂
Compound No. 197: 3O3-B(F)B(F,F)2B(F)-F
Compound No. 198: 4-B(F)B(F,F)2B-F
Compound No. 199: 5-B(F)B(F,F)2B(F)-CL
Compound No. 200: 17-B(F)B(F,F)2B-CL
Compound No. 201: 2-B(F)B(F,F)2B(F)-OCF₃
Compound No. 202: 1-B(F)B(F,F)2B-OCF₃
Compound No. 203: 3-B(F)B(F,F)2B(F)-OCF₂H
Compound No. 204: 4-B(F)B(F,F)2B-OCF₂H
Compound No. 205: 3-B(F)B(F,F)2B(F)-CF₃
   Δε: 13.9, Δn: 0.127, η: 27.7
Compound No. 206: 5-B(F)B(F,F)2B-CF₃
Compound No. 207: 18O-B(F)B(F,F)2B(F)-CF₂H
Compound No. 208: 6-B(F)B(F,F)2B-CF₂H
Compound No. 209: 7-B(F)B(F,F)2B(F,F)-CFH₂
Compound No. 210: 3-B(F,F)B(F,F)2B(F)-F
Compound No. 211: 3-B(F,F)B(F,F)2B-F
Compound No. 212: 3-B(F,F)B(F,F)2B(F)-CL
Compound No. 213: 4-B(F,F)B(F,F)2B-CL
Compound No. 214: 4-B(F,F)B(F,F)2B(F)-OCF₃
Compound No. 215: 4-B(F,F)B(F,F)2B-OCF₃
Compound No. 216: 5-B(F,F)B(F,F)2B(F)-OCF₂H
Compound No. 217: 5-B(F,F)B(F,F)2B-OCF₂H
Compound No. 218: 5-B(F,F)B(F,F)2B(F)-CF₃
Compound No. 219: 2-B(F,F)B(F,F)2B-CF₃
Compound No. 220: 4-B(F,F)B(F,F)2B(F)-CF₂H
Compound No. 221: 6-B(F,F)B(F,F)2B-CF₂H
Compound No. 222: 4-B(F,F)B(F,F)2B(F)-CFH₂
Compound No. 223: 5-B(F)B4B(F,F)-F
Compound No. 224: 5-B(F)B4B(F)-CL
Compound No. 225: 3-B(F)B4B(F,F)-OCF₃
Compound No. 226: 2-B(F)B4B(F)-CF₃
Compound No. 227: 3-BB(F)4B(F)-F
Compound No. 228: 4-BB(F)4B(F)-OCF₃
Compound No. 229: 6-B(F,F)B4B(F,F)-CF₃
Compound No. 230: 5-B(F,F)B4B(F)-OCF₂H
Compound No. 231: 9-B(F)B(F)4B(F)-OCF₃
Compound No. 232: 2-B(F)B(F)4B(F)-CF₃
Compound No. 233: 3-BB(F,F)4B(F,F)-F
Compound No. 234: 1-BB(F,F)4B(F,F)-CL
Compound No. 235: 7-B(F,F)B(F)4B-F
Compound No. 236: 6-B(F,F)B(F)4B-OCF₃
Compound No. 237: 5-B(F)B(F,F)4B(F,F)-OCF₃
Compound No. 238: 4-B(F)B(F,F)4B(F,F)-CF₃
Compound No. 239: 3-B(F,F)B(F,F)4B(F,F)-F
Compound No. 240: 2-B(F,F)B(F,F)4B(F,F)-CL

### Embodiment 7

### Synthesis of (2'-fluoro-4'-propyl-3-fluorobiphenyl-4-yl)methyl=3,4-difluorophenyl=ether (compound (No. 241) represented by general formula (1), where R is C₃H₇, each of Y₁, Y₃, and Y₅ is F, X is F, Z₁ is a covalent bond, and Z₂ is -CH₂O-)

Into the mixture of 0.6 g (13.8 mmol) of NaH and 3 ml of dimethyl formamide (DMF), a solution of 1.8 g (13.8 mmol) of 3,4-difluorophenol dissolved in 20 ml of DMF was added dropwise, and the mixture was stirred for 1 hour. Into this mixture, 3.0 g (9.2 mmol) of 2'-fluoro-4'-propyl-3-fluoro-4-bromomethylbiphenyl was added, and the mixture was heated and refluxed for 4 hours. The reaction mixture was poured into 50 ml of 1N-HCl and extracted by 100 ml of ethyl acetate, and the obtained organic layer was washed twice with a diluted NaHCO₃ solution and three times with water, and dried over anhydrous magnesium sulfate. The solvent was distilled off under reduced pressure, and the residue was subjected to silica-gel-column chromatography (eluent: heptane) to obtain crude (2'-fluoro-4'-propyl-3-fluorobiphenyl-4-yl)methyl 3,4-difluorophenyl ether.

The product was recrystallized from an ethanol/ethyl acetate (9/1) mixed solvent to form the desired compound.

The following compounds Nos. 242 to 278 can be synthesized in the same manner as in Embodiment 7.
Compound No. 242: 3-B(F)CH₂OBB(F,F)-CL
Compound No. 243: 3-B(F)CH₂OBB(F,F)-CF₃
Compound No. 244: 1O5-BCH₂OB(F)B(F)-F
Compound No. 245: 3-BCH₂OB(F)B(F,F)-OCF₂H
Compound No. 246: 3-B(F,F)CH₂OBB(F)-CL
Compound No. 247: 3-B(F,F)CH₂OBB(F,F)-OCF₃
Compound No. 248: 5O-B(F)CH₂OB(F)B(F)-OCF₃
Compound No. 249: 3-B(F)CH₂OB(F)B(F)-CF₃
Compound No. 250: 3-BCH₂OB(F,F)B(F,F)-F
Compound No. 251: 3-BCH₂OB(F,F)B(F)-CL
Compound No. 252: 5O1-B(F,F)CH₂OB(F)B-OCF₃
Compound No. 253: 5-B(F,F)CH₂OB(F)B(F,F)-CF₃
Compound No. 254: 5-B(F)CH₂OB(F,F)B(F,F)-F
Compound No. 255: 5-B(F)CH₂OB(F,F)B(F)-OCF₂H
Compound No. 256: 5-B(F,F)CH₂OB(F,F)B-F
Compound No. 257: 5-B(F,F)CH₂OB(F,F)B(F,F)-OCF₃
Compound No. 258: 5-B(F,F)OCH₂B(F)B(F)-F
Compound No. 259: 3-B(F)BCH₂OB(F,F)-F
Compound No. 260: 5-B(F)BCH₂OB(F)-CL
Compound No. 261: 3-BB(F)CH₂OB(F)-OCF₃
Compound No. 262: 5-BB(F)CH₂OB(F,F)-CF₂H
Compound No. 263: 3-B(F,F)BCH₂OB(F,F)-CL
Compound No. 264: 5-B(F,F)BCH₂OB(F)-CF₃
Compound No. 265: 3-B(F)B(F)CH₂OB(F)-OCF₂H
Compound No. 266: 3-BB(F,F)CH₂OB(F,F)-OCF₃
Compound No. 267: 5-BB(F,F)CH₂OB(F)-CF₂H
Compound No. 268: 3O-B(F,F)B(F)CH₂OB(F,F)-F
Compound No. 269: 5-B(F,F)B(F)CH₂OB-CF₃
Compound No. 270: 3O1-B(F)B(F,F)CH₂OB(F,F)-CL
Compound No. 271: 3-B(F)B(F,F)CH₂OB(F)-CF₃
Compound No. 272: 1O3-B(F,F)B(F,F)CH₂OB(F,F)-F
Compound No. 273: 5-B(F,F)B(F,F)CH₂OB-OCF₃
Compound No. 274: 3-BB(F,F)OCH₂O(F)-CL
Compound No. 275: 5-B(F)2B(F,F)CH₂OB(F)B-OCF₃
Compound No. 276: 5-B(F)CH₂OB(F)2B(F,F)-CF₃
Compound No. 277: 5-B2B(F)OCH₂B(F,F)-F
Compound No. 278: 5-B(F)OCH₂B(F,F)2B(F)-OCF₂H

### Embodiment 8 (Use Example 5)

The properties of the liquid crystal composition of composition example 1 described above are as follows: NI: 88.6, Δε: 8.3, Δn: 0.150, η: 20.5, Vth: 1.73

Although this liquid crystal composition was stored in a freezer of a temperature of -20°C, neither appearance of a smectic phase nor deposition of crystals was observed even after 60 days.

### Embodiment 9 (Use Example 6)

The properties of the liquid crystal composition of composition example 2 described above are as follows:
NI: 78.7, Δε: 8.5, Δn: 0.152, η: 21.8, Vth: 2.23

Although this liquid crystal composition was stored in a freezer of a temperature of -20°C, neither appearance of a smectic phase nor deposition of crystals was observed even after 60 days.

### Embodiment 10 (Use Example 7)

The properties of the liquid crystal composition of composition example 3 described above are as follows:
NI: 90.7, Δε: 29.8, Δn: 0.152, η: 88.0, Vth: 0.94

Although this liquid crystal composition was stored in a freezer of a temperature of -20°C, neither appearance of a smectic phase nor deposition of crystals was observed even after 60 days.

### Embodiment 11 (Use Example 8)

The properties of the liquid crystal composition of composition example 4 described above are as follows:
NI: 84.9, Δε: 7.2, Δn: 0.195, η: 36.5, Vth: 1.96

Although this liquid crystal composition was stored in a freezer of a temperature of -20°C, neither appearance of a smectic phase nor deposition of crystals was observed even after 60 days.

### Embodiment 12 (Use Example 9)

The properties of the liquid crystal composition of composition example 5 described above were as follows:
NI: 62.6, Δε: 10.2, Δn: 0.119, η: 38.4, Vth: 1.52

Although this liquid crystal composition was stored in a freezer of a temperature of -20°C, neither appearance of a smectic phase nor deposition of crystals was observed even after 60 days.

### Embodiment 13 (Use Example 10)

The properties of the liquid crystal composition of composition example 6 described above are as follows:
NI: 72.5, Δε: 9.1, Δn: 0.146, η: 23.3, Vth: 1.35

Although this liquid crystal composition was stored in a freezer of a temperature of -20°C, neither appearance of a smectic phase nor deposition of crystals was observed even after 60 days.

### Embodiment 14 (Use Example 11)

The properties of the liquid crystal composition of composition example 7 described above are as follows:
NI: 76.1, Δε: 23.3, Δn: 0.115, η: 34.3, Vth: 1.09

Although this liquid crystal composition was stored in a freezer of a temperature of -20°C, neither appearance of a smectic phase nor deposition of crystals was observed even after 60 days.

### Embodiment 15 (Use Example 12)

The properties of the liquid crystal composition of composition example 8 described above are as follows:
NI: 90.8, Δε: 5.4, Δn: 0.116, η: 17.8, Vth: 2.17

Although this liquid crystal composition was stored in a freezer of a temperature of -20°C, neither appearance of a smectic phase nor deposition of crystals was observed even after 60 days.

### Embodiment 16 (Use Example 13)

The properties of the liquid crystal composition of composition example 9 described above are as follows: NI: 85.6, Δε: 29.0, Δn: 0.143, η: 43.0, Vth: 0.82

Although this liquid crystal composition was stored in a freezer of a temperature of -20°C, neither appearance of a smectic phase nor deposition of crystals was observed even after 60 days.

### Embodiment 17 (Use Example 14)

The properties of the liquid crystal composition of composition example 10 described above are as follows: NI: 57.1, Δε: 9.8, Δn: 0.113, η: 28.7, Vth: 1.51

Although this liquid crystal composition was stored in a freezer of a temperature of -20°C, neither appearance of a smectic phase nor deposition of crystals was observed even after 60 days.

### Embodiment 18 (Use Example 15)

The properties of the liquid crystal composition of composition example 11 described above are as follows: NI: 64.7, Δε: 7.,3 Δn: 0.154, η: 23.9, Vth: 1.62

Although this liquid crystal composition was stored in a freezer of a temperature of -20°C, neither appearance of a smectic phase nor deposition of crystals was observed even after 60 days.

### Embodiment 19 (Use Example 16)

The properties of the liquid crystal composition of composition example 12 described above are as follows: NI: 95.1, Δε: 5.8, Δn: 0.092, η: 26.2, Vth: 1.96

Although this liquid crystal composition was stored in a freezer of a temperature of -20°C, neither appearance of a smectic phase nor deposition of crystals was observed even after 60 days.

### Embodiment 20 (Use Example 17)

The properties of the liquid crystal composition of composition example 13 described above are as follows: NI: 81.2, Δε: 4.2, Δn: 0.095, η: 20.4, Vth: 2.38

Although this liquid crystal composition was stored in a freezer of a temperature of -20°C, neither appearance of a smectic phase nor deposition of crystals was observed even after 60 days.

### Embodiment 21 (Example 18)

The properties of the liquid crystal composition of composition example 14 described above are as follows: NI: 81.8, Δε: 6.2, Δn: 0.115, η: 25.4, Vth: 1.85

Although this liquid crystal composition was stored in a freezer of a temperature of -20°C, neither appearance of a smectic phase nor deposition of crystals was observed even after 60 days.

### Embodiment 22 (Use Example 19)

The properties of the liquid crystal composition of composition example 15 described above are as follows: NI: 67.1, Δε: 9.4, Δn: 0.088, η: 29.2, Vth: 1.38

Although this liquid crystal composition was stored in a freezer of a temperature of -20°C, neither appearance of a smectic phase nor deposition of crystals was observed even after 60 days.

### Embodiment 23 (Use Example 20)

The properties of the liquid crystal composition of composition example 16 described above are as follows: NI: 71.7, Δε: 13.5, Δn: 0.083, η: 35.5, Vth: 1.25 Although this liquid crystal composition was stored in a freezer of a temperature of -20°C, neither appearance of a smectic phase nor deposition of crystals was observed even after 60 days.

### Embodiment 24 (Use Example 21)

The properties of the liquid crystal composition of composition example 17 described above are as follows: NI: 88.3, Δε: 5.4, Δn: 0.128, η: 21.8, Vth: 2.04

Although this liquid crystal composition was stored in a freezer of a temperature of -20°C, neither appearance of a smectic phase nor deposition of crystals was observed even after 60 days.

### Embodiment 25 (Use Example 22)

The properties of the liquid crystal composition of composition example 18 described above are as follows: NI: 90.1, Δε: 10.0, Δn: 0.115, η: 36.9, Vth: 1.41

Although this liquid crystal composition was stored in a freezer of a temperature of -20°C, neither appearance of a smectic phase nor deposition of crystals was observed even after 60 days.

### Embodiment 26 (Use Example 23)

The properties of the liquid crystal composition of composition example 19 described above are as follows: NI: 80.7, Δε: 5.1, Δn: 0.091, η: 15.5, Vth: 2.24

Although this liquid crystal composition was stored in a freezer of a temperature of -20°C, neither appearance of a smectic phase nor deposition of crystals was observed even after 60 days.

### Embodiment 27 (Use Example 24)

The properties of the liquid crystal composition of composition example 20 described above are as follows: NI: 60.1, Δε: 9.4, Δn: 0.094, η: 27.4, Vth: 1.48

Although this liquid crystal composition was stored in a freezer of a temperature of -20°C, neither appearance of a smectic phase nor deposition of crystals was observed even after 60 days.

### Embodiment 28 (Use Example 25)

The properties of the liquid crystal composition of composition example 21 described above are as follows: NI: 88.7, Δε: 8.1, Δn: 0.134, η: 36.6, Vth: 1.68

Although this liquid crystal composition was stored in a freezer of a temperature of -20°C, neither appearance of a smectic phase nor deposition of crystals was observed even after 60 days.

### Industrial Applicability

The liquid crystalline compounds of the present invention have high voltage holding ratios which have a very small degree of temperature dependence, low threshold voltages which have a very small degree of temperature dependence, high Δn values, and good solubility to other liquid crystal materials even at low temperature. By appropriate selection the substituents and bonding groups of the liquid crystalline compounds of the present invention, a novel liquid crystalline compound having desired properties can be provided.

Therefore, by use of the liquid crystalline compounds of the present invention, there can be provided a novel liquid crystal composition having an extremely high voltage holding ratio which has a very small degree of temperature dependence, low threshold voltages, adequately high Δn and Δε values, and excellent miscibility with other liquid crystal materials. Also, an excellent liquid crystal display element can be provided by use of such a liquid crystal composition.

## Claims

1. A substituted benzene derivative represented by general formula (1), 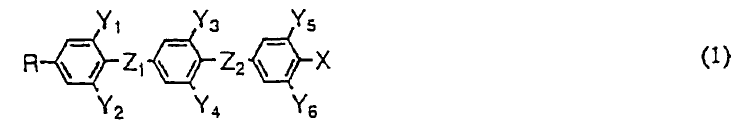 where, R represents a straight chain or branched alkyl group having 1 to 20 carbon atoms, in which each of optional and nonadjacent methylene group (-CH₂-) may be substituted by an oxygen atom; X represents a halogen atom, -CF₃, -CF₂H, -CFH₂, -OCF₃ or -OCF₂H; each of Z₁ and Z₂ independently represents -(CH₂)₂-, -(CH₂)₄-, -CH₂O-, -OCH₂- or a covalent bond, but Z₁ and Z₂ cannot be covalent bonds simultaneously; each of Y₁, Y₂, Y₃, Y₄, Y₅ and Y₆ independently represents H or F, but at least one of Y₁, Y₂, Y₃ and Y₄ represents F; and,
1) when Z₁ = -(CH₂)₂-, Z₂ = a covalent bond, and
a) when Y₁ = F and Y₂ = Y₃ = Y₄ = H,
Y₅ = F,
b) when Y₃ = F and Y₁ = Y₂ = Y₄ = H,
Y₃ = Y₆ = F,
c) when Y₁ = Y₂ = F, Y₃ = Y₄ = H and X = F or -CF₃,
Y₅ = F,
d) when Y₁ = Y₂ = F, Y₃ = Y₄ = H and X = -CF₂H,
Y₆ = H,
e) when Y₁ = Y₃ = F and Y₂ = Y₄ = H,
Y₅ = Y₆ = F or Y₅ = Y₆ = H,
f) when Y₃ = Y₄ = F, Y₁ = Y₂ = H and X = -OCF₃ or -OCF₂H,
Y₅ = F,
g) when Y₃ = Y₄ = F, Y₁ = Y₂ = H and X = -CF₃ or -CF₂H,
Y₅ = Y₆ = F,
h) when Y₁ = Y₂ = Y₃ = F, Y₄ = H and X = F, -OCF₃, -OCF₂H, -CF₃ or -CF₂H,
Y₆ = H,
i) when Y₁ = Y₂ = Y₃ = F, Y₄ = H and X = Cl,
Y₅ = F,
j) when Y₁ = Y₃ = Y₄ = F, Y₂ = H and X = F,
Y₆ = F,
k) when Y₁ = Y₂ = Y₃ = Y₄ = F and X = F, -OCF₃, -OCF₂H, -CF₃ or -CF₂H,
Y₆ = H, and
l) when Y₁ = Y₂ = Y₃ = Y₄ = F and X = Cl,
Y₅ = F,
2) when Z₁ = a covalent bond, Z₂ = -(CH₂)₂-, and
m) when Y₁ = F and Y₂ = Y₃ = Y₄ = H,
Y₅ = F and Y₆ = H,
n) when Y₃ = F and Y₁ = Y₂ = Y₄ = H,
Y₅ = Y₆ = F or Y₅ = Y₆ = H,
o) when Y₁ = Y₃ = F and Y₂ = Y₄ = H,
Y₅ = Y₆ = F or Y₅ = Y₆ = H,
p) when Y₃ = Y₄ = F, Y₁ = Y₂ = H and X = F, Cl, -OCF₃, -OCF₂H or -CF₂H,
Y₆ = H,
q) when Y₁ = Y₃ = Y₄ = F and Y₂ = H,
Y₆ = H,
r) when Y₁ = Y₂ = Y₃ = Y₄ = F,
Y₆ = H, with the proviso that when Z₁ = -(CH₂)₂-, Z₂ = a covalent bond, Y₃ = Y₄ = F and Y₁ = Y₂ = H, X is neither F nor Cl; when Z₁ is a covalent bond, Z₂ is -(CH₂)₂-, Y₁ = F and Y₂ = Y₃ = Y₄ = H, X is neither F, Cl, nor CF₃; and any atom constituting the compound may be substituted by an isomer thereof.

2. A substituted benzene derivative according to Claim 1, wherein Z₁ = -(CH₂)₂-, Z₂ = a covalent bond, Y₁ = Y₅ = F and Y₂ = Y₃ = Y₄ = H.

3. A substituted benzene derivative according to Claim 1, wherein Z₁ = -(CH₂)₂-, Z₂ = a covalent bond, Y₃ = Y₅ = Y₆ = F and Y₁ = Y₂ = Y₄ = H.

4. A substituted benzene derivative according to Claim 1, wherein Z₁ = -(CH₂)₂-, Z₂ = a covalent bond, Y₁ = Y₂ = F, Y₃ = Y₄ = H and X = Cl, -OCF₃ or -OCF₂H.

5. A substituted benzene derivative according to Claim 1, wherein Z₁ = -(CH₂)₂-, Z₂ = a covalent bond, Y₁ = Y₂ = F, Y₃ = Y₄ = Y₆ = H and X = -CF₂H.

6. A substituted benzene derivative according to Claim 1, wherein Z₁ = -(CH₂)₂-, Z₂ = a covalent bond, Y₁ = Y₂ = Y₅ = F, Y₃ = Y₄ = H and X = F or -CF₃.

7. A substituted benzene derivative according to Claim 1, wherein Z₁ = -(CH₂)₂-, Z₂ = a covalent bond, Y₁ = Y₃ = F, Y₂ = Y₄ = H and Y₅ = Y₆ = F or Y₅ = Y₆ = H.

8. A substituted benzene derivative according to Claim 1, wherein Z₁ = -(CH₂)₂-, Z₂ = a covalent bond, Y₃ = Y₄ = Y₅ = F, Y₁= Y₂ = H and X = -OCF₃ or -OCF₂H.

9. A substituted benzene derivative according to Claim 1, wherein Z₁ = -(CH₂)₂-, Z₂ = a covalent bond, Y₃ = Y₄ = Y₅ = Y₆ = F, Y₁ = Y₂ = H and X = -CF₃ or -CF₂H.

10. A substituted benzene derivative according to Claim 1, wherein Z₁ = -(CH₂)₂-, Z₂ = a covalent bond, Y₁ = Y₂ = Y₃ = F, Y₄ = Y₆ = H and X = F, -OCF₃, -OCF₂H, -CF₃ or -CF₂H.

11. A substituted benzene derivative according to Claim 1, wherein Z₁ = -(CH₂)₂-, Z₂ = a covalent bond, Y₁ = Y₂ = Y₃ = Y₅ = F, Y₄= H and X = Cl.

12. A substituted benzene derivative according to Claim 1, wherein Z₁ = -(CH₂)₂-, Z₂ = a covalent bond, Y₁ = Y₃ = Y₄ = F, Y₂ = H and X = Cl, -OCF₃, -OCF₂H, -CF₃ or -CF₂H.

13. A substituted benzene derivative according to Claim 1, wherein Z₁ = -(CH₂)₂-, Z₂ = a covalent bond, Y₁ = Y₃ = Y₄ = F, Y₂ = Y₆ = H and X = F.

14. A substituted benzene derivative according to Claim 1, wherein Z₁ = -(CH₂)₂-, Z₂ = a covalent bond, Y₁ = Y₂ = Y₃ = Y₄ = F, Y₆ = H and X = F, -OCF₃, -OCF₂H, -CF₃ or -CF₂H.

15. A substituted benzene derivative according to Claim 1, wherein Z₁ = -(CH₂)₂-, Z₂ = a covalent bond, Y₁ = Y₂ = Y₃ = Y₄ = Y₅ = F and X = Cl.

16. A substituted benzene derivative according to Claim 1, wherein Z₁ = a covalent bond, Z₂ = -(CH₂)₂-, Y₁ = Y₅ = F, Y₂ = Y₃ = Y₄ = Y₆ = H and X = -OCF₃, -OCF₂H or -CF₂H.

17. A substituted benzene derivative according to Claim 1, wherein Z₁ = a covalent bond, Z₂ = -(CH₂)₂-, Y₃ = F, Y₁ = Y₂ = Y₄ = H and Y₅ = Y₆ = F or Y₅ = Y₆ = H.

18. A substituted benzene derivative according to Claim 1, wherein Z₁ = a covalent bond, Z₂ = -(CH₂)₂-, Y₁ = Y₂ = F and Y₃ = Y₄ = H.

19. A substituted benzene derivative according to Claim 1, wherein Z₁ = a covalent bond, Z₂ = -(CH₂)₂-, Y₁ = Y₃ = F, Y₂ = Y₄ = H and Y₅ = Y₆ = F or Y₅ = Y₆ = H.

20. A substituted benzene derivative according to Claim 1, wherein Z₁ = a covalent bond, Z₂ = -(CH₂)₂-, Y₃ = Y₄ = F, Y₁ = Y₂ = H and X = -CF₃.

21. A substituted benzene derivative according to Claim 1, wherein Z₁ = a covalent bond, Z₂ = -(CH₂)₂-, Y₃ = Y₄ = F, Y₁ = Y₂ = Y₆ = H and X = F, Cl, -OCF₃, -OCF₂H or -CF₂H.

22. A substituted benzene derivative according to Claim 1, wherein Z₁ = a covalent bond, Z₂ = -(CH₂)₂-, Y₁ = Y₂ = Y₃ = F and Y₄ = H.

23. A substituted benzene derivative according to Claim 1, wherein Z₁ = a covalent bond, Z₂ = -(CH₂)₂-, Y₁ = Y₃ = Y₄ = F and Y₂ = Y₆ = H.

24. A substituted benzene derivative according to Claim 1, wherein Z₁ = a covalent bond, Z₂ = -(CH₂)₂-, Y₁ = Y₂ = Y₃ = Y₄ = F and Y₆ = H.

25. A substituted benzene derivative according to Claim 1, wherein Z₁ = -(CH₂)₄-, -CH₂O-, or -OCH₂- and Z₂ = a covalent bond.

26. A substituted benzene derivative according to Claim 1, wherein Z₁ = a covalent bond, and Z₂ = -(CH₂)₄-, -CH₂O- or -OCH₂-.

27. A substituted benzene derivative according to Claim 1, wherein Z₁ = Z₂ = -(CH₂)₂-.

28. A liquid crystal composition containing at least one compound according to any of Claims 1 through 27.

29. A liquid crystal composition containing at least one compound according to any of Claims 1 through 27 as a first component, and at least one compound selected from a group consisting of compounds represented by the following general formulas (2), (3) and (4) as a second component. 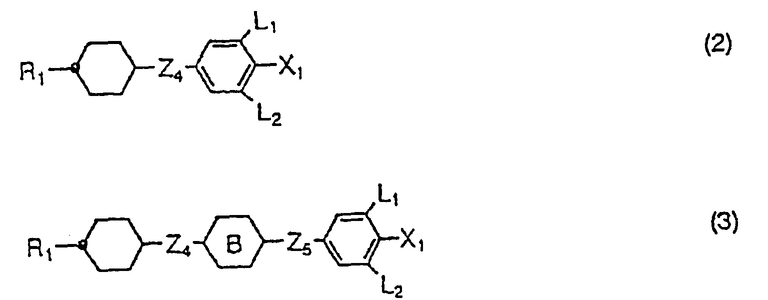 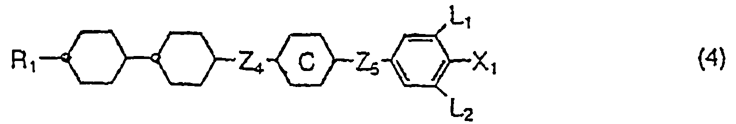 where, R₁ represents an alkyl group having 1 to 10 carbon atoms in which each of optional and nonadjacent methylene groups may be substituted by an oxygen atom or -CH=CH-, and optional hydrogen atoms may be substituted by fluorine atoms; X₁ represents a fluorine atom, a chlorine atom, -OCF₃, -OCF₂H, -CF₃, -CF₂H, -CFH₂, -OCF₂CF₂H or -OCF₂CFHCF₃; each of L₁ and L₂ independently represents a hydrogen atom or a fluorine atom; each of Z₄ and Z₅ independently represents a 1,2-ethylene group, a 1,4-butylene group, -COO-, -CF₂O-, -OCF₂-, -CH=CH- or a covalent bond; ring B represents *trans*-1,4-cyclohexylene, 1,3-dioxane-2,5-diyl, or 1,4-phenylene in which hydrogen atoms may be substituted by fluorine atoms; ring C represents *trans*-1,4-cyclohexylene, or 1,4-phenylene in which hydrogen atoms may be substituted by fluorine atoms; and any atom constituting these compounds may be substituted by an isomer thereof.

30. A liquid crystal composition containing at least one compound according to any of Claims 1 through 27 as a first component, and at least one compound selected from a group consisting of compounds represented by the following general formulas (5) and (6) as a second component. 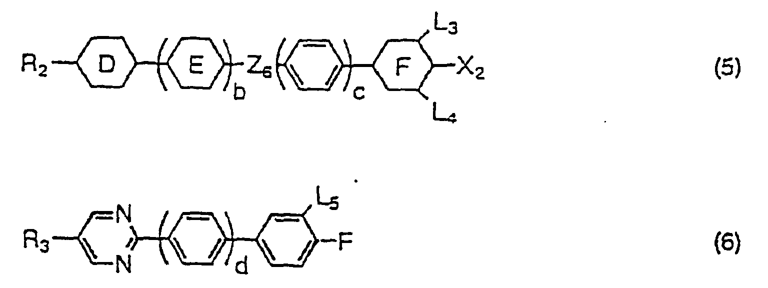 where, each of R₂ and R₃ independently represents an alkyl group having 1 to 10 carbon atoms in which each of optional and nonadjacent methylene groups may be substituted by an oxygen atom or -CH=CH-, and optional hydrogen atoms may be substituted by fluorine atoms; X₂ represents a -CN group or -C≡C-CN; ring D represents *trans*-1,4-cyclohexylene, 1,4-phenylene, 1,3-dioxane-2,5-diyl or pyrimidine-2,5-diyl; ring E represents *trans*-1,4-cyclohexylene, 1,4-phenylene in which hydrogen atoms may be substituted by fluorine atoms or pyrimidine-2,5-diyl; ring F represents *trans*-1,4-cyclohexylene or 1,4-phenylene; Z₆ represents a 1,2-ethylene group, -COO- or a covalent bond; each of L₃, L₄, and L₅ independently represents a hydrogen atom or a fluorine atom; each of b, c and d independently represents 0 or 1; and any atom constituting these compounds may be substituted by an isomer thereof.

31. A liquid crystal composition containing at least one compound according to any of Claims 1 through 27 as a first component, at least one compound selected from a group consisting of compounds represented by the above general formulas (2), (3) and (4) as a second component, and at least one compound selected from a group consisting of compounds represented by the following general formulas (7), (8) and (9) as a third component. 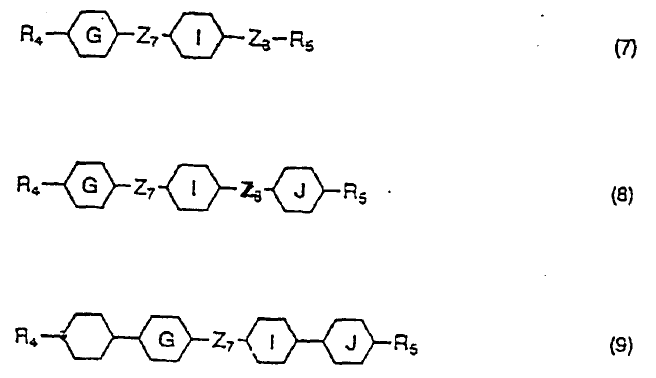 where, each of R₄ and R₅ independently represents an alkyl group having 1 to 10 carbon atoms in which each of optional and nonadjacent methylene groups may be substituted by an oxygen atom or -CH=CH-, and optional hydrogen atoms may be substituted by fluorine atoms; each of ring G, ring I and ring J independently represents *trans*-1,4-cyclohexylene, pyrimidine-2,5-diyl, or 1,4-phenylene in which hydrogen atoms may be substituted by fluorine atoms; each of Z₇ and Z₈ independently represents
-C=C-, -COO-, -CH₂CH₂-, -CH=CH- or a covalent bond; and any atom constituting these compounds may be substituted by an isomer thereof.

32. A liquid crystal composition containing at least one compound according to any of Claims 1 through 27 as a first component, at least one compound selected from a group consisting of compounds represented by the above general formulas (5) and (6) as a second component, and at least one compound selected from a group consisting of compounds represented by the above general formulas (7), (8) and (9) as a third component.

33. A liquid crystal composition containing at least one compound according to any of Claims 1 through 27 as a first component, at least one compound selected from a group consisting of compounds represented by the above general formulas (2), (3) and (4) as a second component, at least one compound selected from a group consisting of compounds represented by the above general formulas (5) and (6) as a third component, and at least one compound selected from a group consisting of compounds represented by the above general formulas (7), (8), and (9) as a fourth component.

34. A liquid crystal composition further containing at least one optically active compound in addition to a liquid crystal composition according to any one of Claims 28 through 33.

35. A liquid crystal element comprising a liquid crystal composition according to any one of Claims 28 through 34.
